(19) Europäisches Patentamt
European Patent Office
Office européen des brevets

(11) Numéro de publication : **0 483 027 A2**

# (12) DEMANDE DE BREVET EUROPEEN

(21) Numéro de dépôt : **91420374.0**

(22) Date de dépôt : **22.10.91**

(51) Int. Cl.[5] : **C07D 471/04,** C07D 409/12, C07D 237/20, A01N 43/653, // (C07D471/04, 249:00, 237:00), (C07D409/12, 333:00, 237:00)

(30) Priorité : **23.10.90 FR 9013338**

(43) Date de publication de la demande : **29.04.92 Bulletin 92/18**

(84) Etats contractants désignés : **AT BE CH DE DK ES FR GB GR IT LI LU NL SE**

(71) Demandeur : **RHONE-POULENC AGROCHIMIE 14-20, rue Pierre Baizet F-69009 Lyon (FR)**

(72) Inventeur : **Cantegril, Richard 31, rue de Lattre de Tassigny F-69009 Lyon (FR)**
Inventeur : **Chene, Alain, Les Terrasses de Saint Rambert 12, chemin de Montpellas F-69009 Lyon (FR)**
Inventeur : **Mortier, Jacques 39, rue Louis Bouquet F-69009 Lyon (FR)**
Inventeur : **Peignier, Raymond 81, bis chemin de Vassieux F-69300 Caluire (FR)**

(74) Mandataire : **Brachotte, Charles et al RHONE-POULENC AGROCHIMIE 14-20, Rue Pierre Baizet B.P. 9163 F-69263 Lyon Cedex 09 (FR)**

(54) **Dérivés de triazolopyridazine, leur procédé de préparation, leur utilisation comme herbicides et leurs intermédiaires.**

(57) L'invention concerne des herbicides de formule (I)

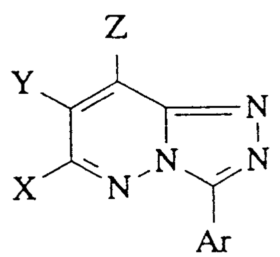

(I)

dans laquelle X représente un halogène, un groupe $R^1$, alkoxy ou un atome d'hydrogène ; Y et Z représentent indépendamment : l'hydrogène, un halogène, un groupe $R^1$, $-OR^1$, $-SR^1$, $-NR^2R^3$ ou cyano ; Ar représente un groupe phényle facultativement substitué ou un hétérocycle penta- ou hexagonal contenant un ou plusieurs hétéro-atomes choisis entre O, S et N, qui est facultativement substitué, $R^1$ est un groupe alkyle à chaîne droite ou ramifiée ; $R^2$ et $R^3$ représentent indépendamment un atome d'hydrogène ou un groupe alkyle à chaîne droite ou ramifiée ; $R^4$ représente un groupe phényle facultativement substitué ; $R^5$ représente un hétérocycle penta- ou hexagonal contenant un ou plusieurs hétéro-atomes O, S ou N ; m a la valeur zéro, 1 ou 2 ; et leurs sels acceptables en agriculture.
L'invention concerne également des compositions herbicides contenant le produit de formule (I).
Application : Procédés de lutte contre des mauvaises herbes au moyen des composés de formule (I) ou de compositions les contenant.



Jouve, 18, rue Saint-Denis, 75001 PARIS

La présente invention concerne des composés herbicides nouveaux de la catégorie des 1,2,4-triazolo-[4,3-b]pyridazines, ainsi que des procédés pour leur préparation, des compositions les contenant et leur utilisation pour lutter contre des mauvaises herbes.

La présente invention a pour objet de proposer des composés qui soient utiles à la fois comme herbicides de pré-levée (ou de pré-germination) et comme herbicides de post-levée (ou de post-germination).

La présente invention a en outre pour objet de proposer des composés qui soient utiles à la fois contre des mauvaises herbes du type monocotylédone et contre des mauvaises herbes du type dicotylédone.

De plus, la présente invention a pour objet de proposer des composés qui soient utiles comme herbicides de pré- et/ou post-levée présentant une sélectivité pour des cultures de plantes monocotylédones (en particulier le blé, le maïs et le riz) et pour des cultures de plantes dicotylédones (en particulier le colza, le soja et le tournesol).

Dans le présent mémoire descriptif, le terme "inférieur" désignant un radical signifie que ce radical ne peut pas avoir plus de 4 atomes de carbone.

La présente invention propose des 1,2,4-triazolo[4,3-b]pyridazines de formule (I) :

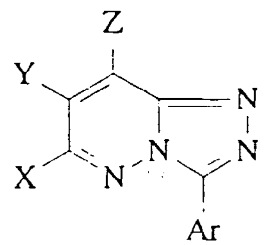

(I)

dans laquelle

X représente un atome d'halogène, un groupe $R^1$, un groupe alkoxy ou un atome d'hydrogène ;

Y et Z représentent indépendamment :

l'hydrogène, un halogène, un groupe $R^1$, $-OR^1$, $-SR^1$, $-NR^2R^3$ ou cyano ;

Ar représente :

un groupe phényle facultativement substitué avec un ou plusieurs groupes qui peuvent être identiques ou différents, choisis entre des groupes $R^1$, $S(O)_mR^1$, $-OR^1$, un groupe cycloalkyle contenant 3 à 6 atomes de carbone, des groupes $-OR^4$, $-OR^5$, $-S(O)_mR^4$, $-NR^2R^4$, $-NR^4COR^1$, $-OCOR^5$, $-S(O)_mR^5$, $-NR^2R^5$, $-NR^5COR^2$ et un atome d'halogène ; ou

un hétérocycle penta- ou hexagonal contenant dans le noyau un ou plusieurs hétéro-atomes choisis entre l'oxygène, le soufre et l'azote, qui est facultativement substitué avec un ou plusieurs groupes qui peuvent être identiques ou différents, choisis entre des groupes $R^1$, $-S(O)_mR^1$, $-OR^1$, un groupe cycloalkyle contenant 3 à 6 atomes de carbone, des groupes $-OR^4$, $-OR^5$, $-S(O)_mR^4$, $-NR^2R^4$, $-NR^4-COR^1$, $-OCOR^5$, $-S(O)_mR^5$, $-NR^2R^5$, $-NR^5COR^2$ et un atome d'halogène ;

$R^1$ représente un groupe alkyle à chaîne droite ou ramifiée contenant 1 à 6 atomes de carbone, facultativement substitué avec un ou plusieurs atomes d'halogènes ;

$R^2$ et $R^3$ représentent indépendamment un atome d'hydrogène ou un groupe alkyle à chaîne droite ou ramifiée contenant jusqu'à 4 atomes de carbone, facultativement substitué avec un ou plusieurs atomes d'halogènes ;

$R^4$ représente un groupe phényle facultativement substitué avec un ou plusieurs groupes choisis entre des groupes $R^1$, $-S(O)_mR^1$, $-OR^1$, $NR^2R^3$, un groupe cycloalkyle contenant 3 à 6 atomes de carbone, et un atome d'halogène ;

$R^5$ représente un hétérocycle penta- ou hexagonal contenant dans le noyau un ou plusieurs hétéro-atomes choisis entre l'oxygène, le soufre et l'azote ;

m a la valeur zéro, 1 ou 2 ; sous réserve que (pour les composés de formule (I) seulement) :

lorsque X représente le chlore et Y et Z représentent chacun l'hydrogène, Ar représente un groupe autre qu'un groupe phényle non substitué, un groupe 3- ou 4-chloro-, méthyl- ou méthoxy-phényle, un groupe 3-méthoxy-4-méthylphényle, un groupe 2,3,4-triméthoxyphényle ou 3,4-dichlorophényle ; et

lorsque X représente l'hydrogène, un groupe méthyle ou méthoxy et Y et Z représentent l'hydrogène, Ar représente un groupe autre qu'un groupe phényle non substitué ;

et leurs sels acceptables en agriculture qui présentent des propriétés herbicides intéressantes.

L'expression "sels acceptables en agriculture" désigne des sels dont les cations ou les anions sont connus et admis dans la pratique pour la formation de sels destinés à être utilisés en agriculture ou en horticulture. De préférence, les sels sont hydrosolubles. Des sels d'addition d'acides convenables des composés de formule

générale (I), qui peuvent renfermer un radical amino, comprennent des sels formés avec des acides inorganiques, par exemple des chlorhydrates, des sulfates, des phosphates et des nitrates, ainsi que des sels formés avec des acides organiques, par exemple l'acide acétique.

Il doit être entendu que, lorsqu'il est fait référence dans la présente invention aux composés de formule générale (I), une telle référence est destinée à désigner également les sels formés avec des acides acceptables en agriculture de composés de formule générale (I), lorsque cela est approprié.

La présente invention propose en outre une catégorie de composés de formule (I), dans laquelle :

* X, Y et Z représentent un atome d'halogène ou un groupe alkyle, halogénalkyle ou alkoxy, ou bien un atome d'hydrogène,

* Ar représente un groupe phényle, facultativement mono- ou polysubstitué (de préférence monosubstitué) avec un groupe alkyle inférieur, alkoxy inférieur ou alkylthio inférieur ou bien un groupe phényle ou un atome d'halogène, de préférence le chlore ou le fluor, ou un hétérocycle Het,

* Het représente un hétérocycle penta- ou hexagonal contenant un hétéro-atome tel que le soufre, l'azote ou l'oxygène, cet hétérocycle étant facultativement mono- ou polysubstitué (de préférence monosubstitué) avec un groupe alkyle inférieur, alkoxy inférieur ou alkylthio inférieur ou bien un atome d'halogène, de préférence le chlore ou le fluor.

Parmi les composés conformes à la présente invention, des composés présentant l'une ou l'autre des caractéristiques suivantes sont préférés :

un seul des trois radicaux X, Y et Z représente un radical autre qu'un atome d'hydrogène,

lorsque X, Y et Z représentent des atomes d'halogènes, ils représentent des atomes de chlore,

lorsque X, Y et Z représentent chacun un radical hydrocarboné, ce radical possède 1 à 4 atomes de carbone, de préférence 1 atome de carbone.

Des composés dans lesquels Ar est monosubstitué et Het représente un radical thiényle sont préférés.

Des composés dans lesquels Z représente un groupe $R^1$ ou un atome d'halogène sont également préférés.

Des composés dans lesquels Z représente un atome d'hydrogène, un atome de chlore ou un groupe méthyle sont également préférés.

Des composés dans lesquels X représente un atome d'hydrogène et Y représente un groupe méthyle sont également préférés.

Les composés suivants peuvent être mentionnés comme composés spécifiques qui peuvent être préparés conformément à la présente invention (voir le système de numérotation des atomes à la fin de la description) :

1) la 3-phényl-s-triazolo-[4,3-b]pyridazine,
2) la 8-méthyl-3-phényl-s-triazolo[4,3-b]pyridazine,
3) la 7-méthyl-3-phényl-s-triazolo-[4,3-b]pyridazine,
4a) la 8-méthyl-3-(3′-méthylthièn-2′-yl)-s-triazolo-[4,3-b]pyridazine,
4b) la 7-méthyl-3-(3′-méthylthièn-2′-yl)-s-triazolo[4,3-b]pyridazine
5a) la 8-méthyl-3-(thièn-3′-yl)-s-triazolo-[4,3-b]pyridazine,
5b) la 7-méthyl-3-(thièn-3′-yl)-s-triazolo-[4,3-b]pyridazine,
6a) la 8-méthyl-3-(2′-fluorophényl)-s-triazolo-[4,3-b]pyridazine,
6b) la 7-méthyl-3-(2′-fluorophényl)-s-triazolo-[4,3-b]pyridazine,
7a) la 8-méthyl-3-(2′,4′-diméthylphényl)-s-triazolo-[4,3-b]pyridazine,
7b) la 7-méthyl-3-(2′,4′-diméthylphényl)-s-triazolo-[4,3-b]pyridazine,
8) la 8-méthyl-3-(thièn-2′-yl)-s-triazolo-[4,3-b]pyridazine,
9) la 7-méthyl-3-(thièn-2′-yl)-s-triazolo-[4,3-b]pyridazine,
10) la 7,8-diméthyl-3-phényl-s-triazolo-[4,3-b]pyridazine,
11) la 8-méthyl-3-(2′-méthylphényl)-s-triazolo-[4,3-b]pyridazine,
12) la 8-méthyl-3-(4′-méthylphényl)-s-triazolo-[4,3-b]pyridazine,
13) la 8-méthyl-3-(3′-méthylphényl)-s-triazolo-[4,3-b]pyridazine,
14) la 8-méthyl-3-(1′-méthylpyrrole-2′-yl)-s-triazolo[4,3-b]pyridazine,
15) la 8-méthyl-3-(4′-isopropoxyphényl)-s-triazolo-[4,3-b]pyridazine,
16a) la 8-méthyl-3-(4′-isopropylphényl)-s-triazolo-[4,3-b]pyridazine,
16b) la 7-méthyl-3-(4′-isopropylphényl)-s-triazolo-[4,3-b]pyridazine,
17) la 8-méthyl-3-(4′-phénoxyphényl)-s-triazolo-[4,3-b]pyridazine,
18) la 8-méthyl-3-(4′-éthylphényl)-s-triazolo-[4,3-b]pyridazine,
19) la 8-méthyl-3-(4′-cyclohexylphényl)-s-triazolo-[4,3-b]pyridazine,
20a) la 8-méthyl-3-(2′-bromophényl)-s-triazolo-[4,3-b]pyridazine,
20b) la 7-méthyl-3-(2′-bromophényl)-s-triazolo-[4,3-b]pyridazine,
21) la 8-méthyl-3-(2′-méthylthiothièn-3′-yl)-s-triazolo-[4,3-b]pyridazine,
22a) la 8-méthyl-3-(3′-phénoxyphényl)-s-triazolo-[4,3-b]pyridazine,

22b) la 7-méthyl-3-(3′-phénoxyphényl)-s-triazolo-[4,3-b]pyridazine,
23) la 8-méthyl-3-[4′-(n-butoxy)phényl]-s-triazolo-[4,3-b]pyridazine
24) la 7-méthyl-3-[3′-(4″-chlorophénoxy)phényl]-s-triazolo-[4,3-b]pyridazine,
25a) la 8-méthyl-3-(3′-fluoro-2′-méthylphényl)-s-triazolo-[4,3-b]pyridazine,
25b) la 7-méthyl-3-(3′-fluoro-2′-méthylphényl)-s-triazolo-[4,3-b]pyridazine,
26) la 7-méthyl-3-(3′-trifluorométhanephényl)-s-triazolo-[4,3-b]pyridazine, et
27) la 7-méthyl-3-(4′-bromophényl)-s-triazolo-[4,3-b]pyridazine.

Les composés de formule générale (I) dans laquelle X représente un atome d'halogène, de préférence le chlore, sont particulièrement utiles comme intermédiaires dans la préparation d'autres composés de formule générale (I), répondant à la définition précitée.

Les composés conformes à la présente invention peuvent être préparés au moyen de différents procédés.

Conformément à une caractéristique de la présente invention, des composés de formule (I) peuvent être préparés au moyen d'un composé du type arylidène-2-(pyridaz-3′-yl)hydrazine, répondant à la formule (II) représentée à la fin de la description, dans laquelle les différents substituants répondent aux définitions précitées, qui est amené à réagir avec un agent oxydant suivant une réaction d'oxydation accompagnée d'une cyclisation. Des sels de cations métalliques dérivés de métaux ayant plusieurs degrés d'oxydation et étant à un haut degré d'oxydation, tels que, par exemple, le tétra-acétate de plomb ou le chlorure ferrique, peuvent être mentionnés comme agents oxydants ; l'oxygène atmosphérique peut également être utilisé comme agent oxydant. La réaction est conduite avantageusement dans un milieu liquide renfermant un solvant organique, le solvant étant de préférence choisi de manière à dissoudre autant que possible les réactifs et les produits finals. Des solvants convenables sont des hydrocarbures, des hydrocarbures halogénés, des acides, des alcools ; des solvants du type des hydrocarbures aromatiques nitrés peuvent également être utilisés, notamment dans le cas où l'agent oxydant est l'oxygène atmosphérique. Le rapport molaire de la quantité d'agent oxydant utilisée à la quantité du composé de formule (II) est généralement compris dans l'intervalle de 1 à 5.

Conformément à une première variante, la réaction d'oxydation du composé de formule (II) peut également être conduite par addition d'un halogène (en une quantité de préférence proche de la proportion stoechiométrique) tel que le brome, puis par une réaction de déshalogénation. Ces réactions d'oxydation (suivant le mode général ou suivant la variante), sont généralement conduites à une température de 10 à 210°C (de préférence de 10 à 50°C pour la variante).

L'oxydation accompagnée d'une cyclisation est effectuée au moyen d'une oxydation-halogénation et d'une réaction de déshalogénation qui est généralement conduite en présence d'un agent alcalin, par exemple en présence d'un sel carboxylique d'un métal alcalin, tel que l'acétate de sodium dans un milieu consistant en acide acétique. Le rapport molaire de la quantité de l'agent alcalin utilisé à la quantité du composé halogéné de formule (II) est généralement compris dans l'intervalle de 1 à 5.

Conformément à une deuxième variante, la réaction d'oxydation du composé de formule (II) peut également être conduite par addition d'un N-halogéno-N-métallosulfonamidate de formule (IV) : $RSO_2N^-XM^+$, dans laquelle R représente un groupe alkyle ou, de préférence, un groupe phényle facultativement monosubstitué en position para avec un groupe alkyle (par exemple un groupe p-tolyle), X représente un atome d'halogène (de préférence le chlore ou le brome) et M représente un atome de métal alcalin (de préférence le sodium). Un exemple apprécié de N-halogéno-N-métallosulfonamidate est la chloramine T, de formule (IVa) :

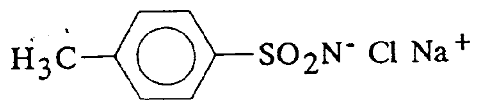

(IVa)

L'utilisation d'un N-halogéno-N-métallosulfonamidate répondant à la formule précitée comme agent d'oxydation cyclisante pour les arylidène-2-(pyridaz-3′-yl)hydrazines de formule générale (II) est nouvelle et constitue ainsi un procédé nouveau pour la préparation des composés de formule (I).

Cette réaction d'oxydation cyclisante est généralement conduite à une température de 10 à 150°C (de préférence de 20 à 50°C) et est avantageusement conduite dans un milieu liquide consistant en un solvant organique, de préférence dans des alcools.

Le rapport molaire de la quantité de l'agent oxydant utilisé à la quantité du composé de formule (II) est de préférence proche du rapport stoechiométrique.

Conformément à une autre caractéristique de la présente invention, un deuxième procédé pour la préparation des composés de formule (I) consiste en la déshydratation d'un composé du type 3-(aroylhydrazino)pyridazine répondant à la formule (III) représentée à la fin de la description, dans laquelle les différents substituants répondent aux définitions mentionnées en rapport avec la formule (I) (déshydratation cyclisante).

La réaction de déshydratation cyclisante du composé de formule (III) est conduite de manière usuelle par chauffage à une température de 100 à 250°C, avec élimination de l'eau formée lors de la progression de la réaction. L'eau formée peut être éliminée par une distillation simple ou par une distillation azéotrope si le chauffage est conduit en présence d'un solvant aromatique capable de dissoudre le composé de formule (III) et de former un azéotrope avec l'eau. Des hydrocarbures aromatiques, halogénés ou non, ainsi que des phénols, par exemple le xylène, le phénol et le 1,2,4-trichlorobenzène, peuvent être mentionnés comme solvant azéotrope convenable pour ce mode de déshydratation.

Cependant, le procédé peut également être mis en oeuvre dans d'autres solvants organiques inertes tels que, par exemple, le dioxanne.

Conformément à une autre caractéristique de la présente invention, un troisième procédé de préparation des produits de formule (I) consiste à faire réagir un composé du type hydrazine répondant à la formule (V), telle qu'elle est représentée à la fin de la description, avec un composé de formule (VI) :

$U_1$-C(=$W_1$)-Ar suivant la réaction :

$$(V) + (VI) \rightarrow (I) + U_1H + W_1H_2$$

les différents radicaux dans ces formules (V) et (VI) répondant aux définitions indiquées en rapport avec la formule (I), et, en outre :

$W_1$ représente un atome d'oxygène ou un groupe NH,

lorsque $W_1$ représente un atome d'oxygène, $U_1$ représente alors un groupe hydroxyle [dans ce cas, le composé (VI) est un acide] ou un groupe alkoxy [dans ce cas, le composé (VI) est un ester] ou un groupe aroyloxy [dans ce cas, le composé (VI) est un anhydride d'acide] ou un atome d'halogène [dans ce cas, le composé (VI) est un halogénure d'acide], de préférence le chlore,

lorsque $W_1$ représente un groupe NH, $U_1$ représente alors un groupe alkoxy, alkylthio ou arylalkylthio.

La réaction précédente est conduite à une température généralement comprise dans l'intervalle de 20 à 200°C, de préférence de 50 à 180°C, pendant un temps de 1 à 24 heures. Les réactifs (V) et (VI) sont utilisés en un rapport molaire de 0,8 à 1,2, respectivement, de préférence égal à 1 ou proche de cette valeur. La réaction peut être conduite en la présence ou en l'absence d'un solvant. Des solvants conduisant à la formation de mélanges azéotropes peuvent être utilisés comme solvant, de la manière indiquée ci-après, dans certains cas. Il est possible également d'utiliser des solvants du type alcool lorsque le composé de formule (VI) est un imidate, ou bien un solvant aromatique tel qu'un hydrocarbure, halogéné ou non, ou la pyridine lorsque le composé de formule (VI) est un thioimidate ou un halogénure d'acide.

Les produits légers de la réaction ($U_1H$ et $W_1H_2$) sont éliminés avantageusement lors de la progression de la réaction, généralement par distillation lorsqu'il s'agit d'eau ou d'alcools (dans ce cas, $U_1$ représente un groupe hydroxy ou alkoxy), ou par dégazage ou piégeage au moyen d'une base tertiaire telle que la triéthylamine lorsque $U_1$ représente un atome d'halogène, ou bien par piégeage de la molécule d'ammoniac lorsque $W_1$ représente un groupe NH et $U_1$ représente un groupe alkoxy (dans ce cas, le produit de formule (VI) est un imidate).

La distillation précitée de produits légers peut être une distillation azéotrope conduite en présence de solvants aromatiques halogénés ou non halogénés tels que la pyridine ou des chlorobenzènes, en particulier le 1,2,4-trichlorobenzène.

Le piégeage précité de l'ammoniac est avantageusement conduit en présence d'acides (de préférence carboxyliques) ou de leurs dérivés tels que des anhydrides d'acides ou des halogénures d'acides. Ces acides ou leurs dérivés sont utilisés en des quantités molaires généralement égales à une valeur de 1 à 30 fois la quantité de produit de formule (VI) utilisée. Ces proportions sont du même ordre (mutatis mutandis) dans le cas où une base est utilisée, lorsque $U_1$ représente un atome d'halogène.

Conformément à une autre caractéristique de la présente invention, un quatrième procédé de préparation de composés de formule (I) dans laquelle X représente un atome d'hydrogène consiste en l'hydrogénolyse de composés de formule (I) dans laquelle X représente un atome d'halogène (de préférence le chlore), pour le remplacement de l'atome d'halogène par un atome d'hydrogène.

Cette réaction d'hydrogénolyse est conduite avantageusement dans un milieu liquide à base d'un solvant organique, le solvant étant de préférence choisi de manière à dissoudre autant que possible les réactifs et les produits finals.

Des solvants convenables sont des alcools et le dioxanne. Cette réaction d'hydrogénolyse est généralement conduite à une température de 10 à 100°C (de préférence de 20 à 30°C) en présence d'un agent accepteur d'acide, tel que la triéthylamine, l'hydroxyde d'ammonium ou la collidine, et d'un catalyseur (de préférence le palladium sur du carbone) suivant un procédé similaire à ceux décrits par R.N. CASTLE dans The Chemistry of Heterocyclic Compounds, Pyridazines, édité en 1973 par Interscience/Wiley, Volume 28, pages 245-248.

Conformément à une autre caractéristique de la présente invention, des composés de formule (I), dans laquelle Z représente le chlore ou le brome et X et Y représentent l'hydrogène ou un groupe $R^1$, peuvent être

préparés par réaction d'un composé de formule (IX) :

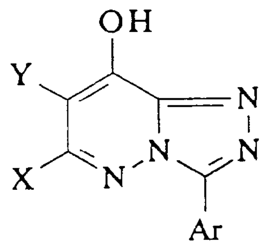

(IX)

dans laquelle X et Y représentent l'hydrogène ou un groupe $R^1$, avec un agent d'halogénation pour le remplacement du groupe hydroxy par un atome de chlore ou de brome. En général, l'oxychlorure de phosphore ou l'oxybromure de phosphore est utilisé. La réaction peut être conduite facultativement en présence d'un solvant inerte, tel que le toluène ou le chloroforme, à des températures allant de la température ambiante à la température de reflux du mélange.

Conformément à une autre caractéristique de la présente invention, des composés de formule (I), dans laquelle Z représente le fluor et X et Y représentent l'hydrogène ou un groupe $R^1$, peuvent être préparés par réaction d'un composé de formule (I), dans laquelle Z représente le chlore ou le brome, et X et Y représentent l'hydrogène ou un groupe $R^1$, avec un sel de formule M-F dans laquelle M représente un métal alcalin ou alcalino-terreux. En général, M représente le sodium, le potassium ou le césium. La réaction est généralement conduite dans un solvant tel que le sulfolane à une température de 50 à 200°C.

Conformément à une autre caractéristique de la présente invention, des composés de formule (I), dans laquelle Z représente un groupe $-OR^1$, $-SR^1$, $-NR^2R^3$ ou cyano, peuvent être préparés à partir de composés de formule (I), dans laquelle Z représente un atome d'halogène, par déplacement nucléophile de l'atome d'halogène par le réactif nucléophile approprié, tel qu'un réactif de formule $M-SR^1$, $M-OR^1$, $H-NR^2R^3$ ou M-CN dans laquelle M représente un cation de métal alcalin. La réaction est généralement conduite dans un solvant tel que le diméthylformamide, le diméthylsulfoxyde, un alcool ou une solution aqueuse d'un alcool, à des températures de 25 à 150°C.

Conformément à une autre caractéristique de la présente invention, des composés de formule (I), dans laquelle Z représente un groupe $-OR^1$, peuvent être préparés par réaction d'un composé de formule (IX) avec un agent d'alkylation tel qu'un agent de formule $R^1{}_2SO_4$ ou $R^1$-L, dans laquelle L représente un atome d'halogène ou un alkyl- ou arylsulfonate, pour le remplacement du groupe hydroxy par un groupe $-OR^1$. La réaction est généralement conduite dans un solvant inerte tel que le tétrahydrofuranne, un alcool ou une solution aqueuse d'un alcool à une température comprise dans l'intervalle de la température ambiante à 100°C. De préférence, une base, telle que l'hydroxyde de sodium ou la triéthylamine, est présente. Lorsque $R^1$ représente un groupe halogénalkyle, L représente un atome d'halogène, de préférence le chlore.

Des sels des composés de formule (I) peuvent être préparés par des procédés connus, par exemple par traitement d'un composé de formule (I) avec l'acide approprié.

La présente invention concerne également des produits nouveaux qui sont utiles en particulier comme intermédiaires dans la préparation des composés de formule (I), caractérisés en ce qu'ils répondent à l'une des formules (II), (III), (V) et (VIII) représentées ci-après, dans lesquelles les symboles X, Y, Z et Ar répondent aux définitions mentionnées pour la formule (I), sous réserve que :

i) pour des composés de formule (II) :

dans laquelle X représente un groupe 1-méthyl-1-éthylpropyle et Y et Z représentent chacun l'hydrogène, Ar ne représente pas un groupe 4-n-butoxyphényle, 2,4-, 2,5- ou 3,5-diméthylphényle, 3-bromophényle, 2,4- ou 3,5-dichlorophényle, 2- ou 4-chlorophényle ou 2,4-diméthoxyphényle,

lorsque X, Y et Z représentent chacun un groupe méthyle, Ar ne représente pas un groupe phényle non substitué,

lorsque X et Z représentent chacun un groupe méthyle et Y représente l'hydrogène, Ar ne représente pas un groupe phényle non substitué ou substitué seulement avec le chlore,

lorsque X représente le chlore et Y et Z représentent chacun l'hydrogène, Ar ne représente pas un groupe phényle non substitué ou substitué seulement avec le chlore ou un groupe méthoxy ;

ii) pour des composés de formule (V)

dans laquelle Y et Z représentent chacun un groupe méthyle, X ne représente pas le chlore ; et

iii) pour des composés de formule (VIII) :

lorsque T représente le chlore et Y et Z représentent chacun un groupe méthyle, X ne représente

pas l'hydrogène ou le chlore,

lorsque Y et Z représentent chacun l'hydrogène, X ne représente pas un groupe méthyle, butyle, trichlorométhyle ou un halogène,

lorsque T et Y représentent chacun le chlore, X et Z représentent des groupes autres que le chlore.

Dans ce qui suit, mais également dans ce qui précède, les réactions conduites à haute température dans un solvant, sauf spécifications contraires, sont conduites avantageusement à la température d'ébullition du solvant indiqué.

La préparation d'arylidène-2-(pyridaz-3'-yl)hydrazines de formule (II) est aisément mise en oeuvre par réaction de 3-hydrazinopyridazines de formule (V) avec des aldéhydes de formule Ar-CHO, formules dans lesquelles les symboles X, Y, Z et Ar répondent aux définitions indiquées pour la formule (I). La réaction est généralement conduite à une température comprise dans l'intervalle de 50 à 150°C, de préférence dans un solvant. Un alcool inférieur, tel que le méthanol ou l'éthanol, est utilisé avantageusement comme solvant. La réaction est accrue par la présence de quantités catalytiques d'un acide inorganique ou organique, par exemple l'acide chlorhydrique, sulfurique, acétique, trichloracétique, perchlorique ou p-toluènesulfonique. Une telle réaction est décrite dans Quaterly Review, Chemical Society, Volume 23, pages 37-56, 1969, dans l'article J. BUCKINGHAM, ainsi que dans Houben-Weyl, Methoden der organischen Chemie, 4$^{ème}$ édition, 1967, Volume X-2, pages 410-487.

La préparation de 3-(aroylhydrazino)pyridazines de formule (III) est aisément mise en oeuvre par réaction de 3-hydrazinopyridazines de formule (V) avec les composés de formule (VII) de formule Ar-CO-$U_2$, formules dans lesquelles les symboles X, Y, Z et Ar répondent aux définitions mentionnées pour la formule (I) et $U_2$ répond à l'une des définitions mentionnées précédemment pour $U_1$. La réaction s'effectue conformément au schéma :

$$(V) + (VII) \rightarrow (III) + U_2H$$

La réaction est généralement conduite par mélange des réactifs à une température comprise dans l'intervalle de 0 à 180°C en la présence ou en l'absence d'un solvant. Un solvant polaire est utilisé avantageusement comme solvant.

Comme solvants qui peuvent être utilisés plus particulièrement, on peut mentionner des alcools, lorsque le composé de formule Ar-CO-$U_2$ n'est pas un halogénure d'acide ; des éthers ou des hydrocarbures aliphatiques chlorés ou non chlorés tels que le chlorure de méthylène et le chloroforme ; des solvants ayant les propriétés d'accepteurs d'acides, tels que la pyridine, peuvent également être utilisés lorsque le composé de formule Ar-CO-$U_2$ représente un halogénure d'acide.

La rapport des deux réactifs [celui de formule (V) et celui de formule (VII)] peut varier dans de larges limites de part et d'autre du rapport stoechiométrique. Lorsque le composé de formule Ar-CO-$U_2$ est un acide ($U_2$ représente un groupe OH), un excès du dernier dérivé est utilisé par rapport au composé de formule (V), par exemple 2 à 8 moles par mole de réactif de formule (V). Lorsque le composé de formule Ar-CO-$U_2$ est un ester ($U_2$ représente un groupe alkoxy), une quantité, permettant de parvenir à un rapport pratiquement stoechiométrique, de ce dernier dérivé est utilisée par rapport à la quantité du composé de formule (V), par exemple 0,8 à 1,1 mole par mole de composé de formule (V). Lorsque le composé de formule Ar-CO-$U_2$ est un halogénure d'acide ($U_2$ représente un atome d'halogène), un excès du composé de formule (V) est utilisé par rapport à la quantité du composé de formule (VII), par exemple 1 à 5 moles par mole de composé de formule Ar-CO-$U_2$.

Des procédés dont la mise en oeuvre permet d'obtenir les composés de formule (III), conformément à la description précédente, sont décrits dans l'ouvrage de PATAI, The Chemistry of Carboxylic Acids and Esters, Volume 5, Chapitre 9, pages 425-428, édité en 1969 par Interscience/Wiley, dans l'article de SATCHELL, ainsi que dans Houben-Weyl, Methoden der organischen Chemie, 1952, Volume VIII, Chapitre 5, pages 676-680.

La préparation de 3-(aroylhydrazino)pyridazines de formule (III) peut également être aisément mise en oeuvre par réaction d'un dérivé de pyridazine de formule (VIII) avec un arylhydrazide de formule Ar-CO-NH-$NH_2$, formules dans lesquelles les symboles X, Y, Z et Ar répondent aux définitions mentionnées précédemment pour les formules (I) et (III) et T représente un atome d'halogène, de préférence le chlore ou le brome. La réaction s'effectue conformément au schéma :

$$(VIII) + ArCONHNH_2 \rightarrow (III) + HT$$

Cette réaction est conduite avantageusement à une température comprise dans l'intervalle de 50 à 150°C dans des solvants tels que des alcools ou des solvants aromatiques, par exemple la pyridine ou des hydrocarbures tels que le toluène. Le rapport des réactifs est avantageusement proche du rapport stoechiométrique, égal par exemple à un rapport molaire allant de 0,8 à 1,2. La réaction peut être accrue par la présence d'une quantité, permettant également de parvenir à un rapport proche du rapport stoechiométrique, d'un agent basique tel qu'un alcoolate ou un bicarbonate de métal alcalin.

Les arylhydrazides de formule Ar-CO-NH-$NH_2$ peuvent être obtenus par réaction d'un hydrate d'hydrazine avec un acide ou un de ses dérivés tels que des esters, des halogénures ou des anhydrides. La réaction est

conduite avantageusement à une température comprise dans l'intervalle de 0 à 150°C, de préférence en présence d'un solvant tel qu'un alcool inférieur, le rapport molaire de l'hydrate d'hydrazine à l'autre réactif étant généralement compris dans l'intervalle de 1,01 à 1,5.

Des procédés dont la mise en oeuvre rend possible l'obtention des arylhydrazides conformes à la description précédente sont décrits dans le manuel de PATAI, The Chemistry of Amides Volume 11, Chapitre 10, pages 515-600, édité en 1970 par Interscience/Wiley dans un article de PAULSEN et STOYE, ainsi que dans Organic Reactions, the CURTIUS reaction, 1962, Volume III, Chapitre 9, pages 366-369 édité par Wiley.

Les 3-hydrazinopyridazines de formule (V) peuvent être préparées en particulier par un procéeé similaire à ceux décrits par R.N. CASTLE dans The Chemistry of Heterocyclic Compounds, Pyridazines, édité en 1973 par Interscience/Wiley, Volume 28, pages 254-256.

En ce qui concerne les 3-halogénopyridazines de formule (VIII), ces composés peuvent être préparés par un procédé similaire à ceux décrits par R.N. CASTLE, dans le même manuel, pages 221-242.

Les composés de formule (IX) peuvent être préparés par réaction d'un composé de formule (X) :

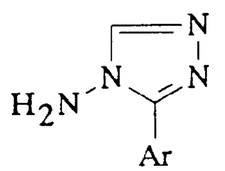

(X)

avec un composé de formule (XI) ou (XII) :

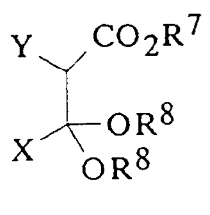

(XI)

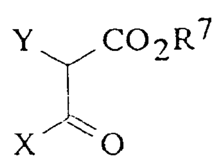

(XII)

formules dans lesquelles X représente l'hydrogène ou un groupe $R^1$ et $R^7$ et $R^8$ représentent un groupe alkyle. La réaction est généralement conduite à une température comprise dans l'intervalle de 100 à 200°C, facultativement en présence d'un solvant tel que l'acide acétique. Cette réaction est décrite dans la littérature (J. Am. Chem. Soc. 81, 6289 (1959).

Les composés de formule (V), dans laquelle X représente l'hydrogène, peuvent être préparés à partir du composé correspondant de formule (V), dans laquelle X représente un halogène, par réaction avec l'hydrate d'hydrazine en présence d'un catalyseur consistant en palladium sur du charbon. La réaction est généralement conduite en présence d'un solvant tel que le méthanol à une température comprise dans l'intervalle de 25°C à la température de reflux du mélange.

Des composés de formule (V) dans laquelle Z représente un groupe $-OR^1$ et X et Y représentent l'hydrogène, peuvent être préparés par réaction d'un composé de formule (XIII) :

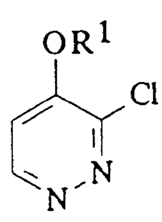

(XIII)

avec l'hydrate d'hydrazine. La réaction est conduite facultativement en présence d'un solvant, par exemple l'éthanol, à une température de 25 à 100°C.

Des composés de formule (XIII) peuvent être préparés par réaction d'un composé de formule (XIV) :

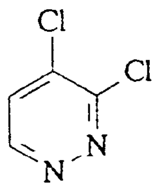

(XIV)

avec un composé de formule $R^1O$-Na en présence d'un solvant inerte à une température de 25 à 150°C.

Des composés de formule (IX) peuvent être préparés par réaction de composés de formule (XV) :

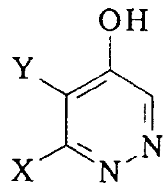

(XV)

avec un composé de formule (VI) répondant à la définition précitée. La réaction est conduite de la manière décrite précédemment pour la préparation d'un composé de formule (I) à partir de composés de formules (V) et (VI).

Des composés de formule (XV) peuvent être préparés par réaction d'un composé de formule (XVI) :

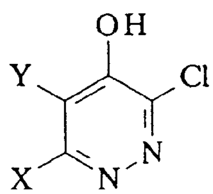

(XVI)

avec l'hydrate d'hydrazine. La réaction est conduite facultativement en présence d'un solvant, tel que l'éthanol, à une température comprise dans l'intervalle de 25°C à la température de reflux du mélange.

Les exemples suivants, proposés à titre non limitatif, illustrent la présente invention et montrent comment elle peut être mise en pratique.

Les Exemples 1 à 9 illustrent la préparation de composés herbicides conformes à la présente invention. Les Exemples I-1 à I-13 illustrent la préparation de composés intermédiaires des produits précédents.

***Exemple 1 :**

De la 6-chloro-3-phényl-s-triazolo[4,3-b]pyridazine (0,75 g, 0,0033 mole) (préparée de la manière indiquée dans l'Exemple I-1), 10 % de palladium sur du carbone (0,085 g) et une solution à 28 % d'hydroxyde d'ammonium (3 ml) dans du méthanol (60 ml) sont agités pendant 4 heures à température ambiante sous une atmosphère d'hydrogène à une pression de 3 bars. Après filtration, le résidu est chromatographié (éluant heptane/acétate d'éthyle dans le rapport 50/50). De la 3-phényl-s-triazolo[4,3-b]pyridazine (0,35 g, 0,0018 mole) est obtenue ; P.F. = 145°C (rendement 55 %).

***Exemple 2 :**

L'Exemple 1 est répété, avec remplacement de la 6-chloro-3-phényl-s-triazolo[4,3-b]pyridazine (0,75 g) par de la 6-chloro-8-méthyl-3-phényl-s-triazolo-[4,3-b]pyridazine (1 g, 0,0041 mole) (préparée de la manière indiquée dans l'Exemple I-2), le procédé étant mis en oeuvre sous une atmosphère d'hydrogène à une pression de 3,15 bars pendant 6 heures. De la 8-méthyl-3-phényl-s-triazolo-[4,3-b]pyridazine (0,59 g, 0,0028 mole) est obtenue ; P.F. = 51°C (rendement 70 %).

***Exemple 3 :**

L'Exemple 1 est répété, avec remplacement de la 6-chloro-3-phényl-s-triazolo-[4,3-b]pyridazine (0,75 g) par de la 6-chloro-7-méthyl-3-phényl-s-triazolo-[4,3-b]pyridazine (0,7 g, 0,0029 mole) (préparée de la manière indiquée dans l'Exemple I-2), le procédé étant mis en oeuvre sous une atmosphère d'hydrogène à une pression de 2,5 bars pendant un temps de 3 heures 30 minutes. De la 7-méthyl-3-phényl-s-triazolo-[4,3-b]pyridazine (0,47 g, 0,0022 mole) est obtenue ; P.F. = 180°C (rendement 76 %).

***Exemple 4 :**

Composés 4a, 4b, 10, 11, 12, 13, 14, 16a, 16b, 17, 18, 20a, 20b, 21, 22a, 22b, 24, 25a, 25b, 27.

De la chloramine T (2,12 g, 0,0075 mole) est ajoutée rapidement à température ambiante à une solution éthanolique (50 ml) d'un mélange de 3-méthyl-2-thiophène-carboxaldéhyde-(4'/5'-méthylpyridaz-3'-yl)hydrazones (1,75 g, 0,0075 mole, préparées de la manière indiquée dans l'Exemple I-3). Le mélange réactionnel est agité pendant 10 minutes. Après évaporation du solvant, le résidu est chromatographié (éluant heptane/acétate d'éthyle dans le rapport 20/80). De la 8-méthyl-3-(3'-méthylthièn-2'-yl)-s-triazolo-[4,3-b]pyridazine (4a) (0,49 g, 0,0021 mole), P.F. = 170°C, et de la 7-méthyl-3-(3'-méthylthièn-2'-yl)s-triazolo-[4,3-b]pyridazine (4b) (0,75 g, 0,0033 mole), P.F. = 191°C, sont obtenues (rendement 71 %).

En procédant d'une manière similaire, on a préparé les composés suivants de formule générale (I) à partir des matières de départ substituées de la manière appropriée :

| Composé N° | Z | Y | X | Ar | P.F. |
|---|---|---|---|---|---|
| 10 | Me | Me | H | Phényle | 170°C |
| 11 | Me | H | H | 2-Méthylphényle | 133°C |
| 12 | Me | H | H | 4-Méthylphényle | 170°C |
| 13 | Me | H | H | 3-Méthylphényle | 117°C |
| 14 | Me | H | H | 1-Méthylpyrrole-2-yle | 182°C |
| 16a | Me | H | H | 4-Isopropylphényle | 105°C |
| 16b | H | Me | H | 4-Isopropylphényle | 121°C |
| 17 | Me | H | H | 4-Phénoxyphényle | 156°C |
| 18 | Me | H | H | 4-Ethylphényle | 115°C |
| 20a | Me | H | H | 2-Bromophényle | 188°C |
| 20b | H | Me | H | 2-Bromophényle | 196°C |
| 21 | Me | H | H | 2-Méthylthiothiène-3-yle | 135°C |
| 22a | Me | H | H | 3-Phénoxyphényle | 102°C |
| 22b | H | Me | H | 3-Phénoxyphényle | 133°C |
| 24 | H | Me | H | 3-(4'-Chlorophénoxy)phényle | 165°C |
| 25a | Me | H | H | 3-Fluoro-2-méthylphényle | 195°C |
| 25b | H | Me | H | 3-Fluoro-2-méthylphényle | 201°C |
| 27 | H | Me | H | 4-Bromophényle | 238°C |

***Exemple 5 :**

L'Exemple 4 est répété, avec remplacement des 3-méthyl-2-thiophènecarboxaldéhyde-(4'/5'-méthylpyridaz-3'-yl)hydrazones (1,75 g) par un mélange de 3-thiophènecarboxaldéhyde-(4'/5'-méthylpyridaz-3'-yl)hydrazones (2,2 g, 0,01 mole) (préparées de la manière indiquée dans l'Exemple I-4). De la chloramine T (2,82 g, 0,01 mole) est utilisée dans ce cas. Après chromatographie (éluant acétate d'éthyle), de la 8-méthyl-3-(thiène-3'-yl)-s-triazolo-[4,3-b]pyridazine (5a) (0,65 g, 0,003 mole), P.F. = 156°C, et de la 7-méthyl-3-(thiène-3'-yl)-s-triazolo-[4,3-b]pyridazine (5b) (0,78 g, 0,0036 mole), P.F. = 151°C, sont obtenues (rendement 66 %).

***Exemple 6 :**

Composés 6a, 6b, 15, 19, 23, 26.

Un mélange de 3-hydrazino-4/5-méthylpyridazines (1,25 g, 0,01 mole) (préparées de la manière indiquée dans l'Exemple I-5) et de 2-chlorure de 2-fluorobenzoyle (1,59 g, 0,01 mole) dans du toluène (50 ml) est chauffé

pendant 2 heures dans un ballon à fond rond muni d'un condenseur et équipé d'un séparateur de Dean Stark. Après évaporation du solvant et chromatographie du résidu (éluant acétate d'éthyle), de la 8-méthyl-3-(2′-fluorophényl)-s-triazolo-[4,3-b]pyridazine (6a) (0,26 g, 0,0011 mole), P.F. = 126°C, et de la 7-méthyl-3-(2′-fluorophényl)-5-triazolo-[4,3-b]pyridazine (6b) (0,10 g, 0,00043 mole), P.F. = 182°C, sont obtenues (rendement 15 %).

Par remplacement du toluène par le 1,4-dioxanne et sans l'utilisation d'un séparateur de Dean Stark, les composés suivants de formule générale (I) ont été préparés à partir des matières de départ substituées de la manière appropriée :

| Composé N° | Z | Y | X | Ar | P.F. |
|---|---|---|---|---|---|
| 15 | Me | H | H | 4-Isopropoxyphényle | 155°C |
| 19 | Me | H | H | 4-Cyclohexylphényle | 226°C |
| 23 | Me | H | H | 4-(n-Butoxy)phényle | 144°C |
| 26 | H | Me | H | 3-Trifluorométhylphényle | 147°C |

***Exemple 7 :**

L'Exemple 4 est répété, avec remplacement des 3-méthyl-2-thiophènecarboxaldéhyde-(4′/5′-méthylpyridaz-3′-yl)hydrazones (1,75 g) par un mélange de 2,4-diméthylbenzaldéhyde-(4′/5′-méthylpyridaz-3′-yl)hydrazones (2,10 g, 0,0088 mole) (préparées de la manière indiquée dans l'Exemple I-6). De la chloramine T (2,45 g, 0,0087 mole) est utilisée dans ce cas. Après chromatographie (éluant heptane/acétate d'éthyle dans le rapport 20/80), de la 8-méthyl-3-(2′,4′-diméthylphényl)-s-triazolo-[4,3-b]pyridazine (7a) (0,51 g, 0,0021 mole), P.F. = 156°C, et de la 7-méthyl-3-(2′,4′-diméthylphényl)-s-triazolo-[4,3-b]pyridazine (7b) (0,95 g, 0,004 mole), P.F. = 144°C, sont obtenues (rendement 71 %).

***Exemple 8 :**

L'Exemple 4 est répété, avec remplacement des 3-méthyl-2-thiophènecarboxaldéhyde-(4′/5′-méthylpyridaz-3′-yl)hydrazones (1,75 g) par de la 2-thiophènecarboxaldéhyde-(4′-méthylpyridaz-3′-yl)hydrazone (1,4 g, 0,0064 mole) (préparée de la manière indiquée dans l'Exemple I-7). De la chloramine T (1,80 g, 0,0064 mole) est utilisée dans ce cas. Après chromatographie (éluant acétate d'éthyle), de la 8-méthyl-3-(thiène-2′-yl)-s-triazolo[4,3-b]pyridazine (0,28 g, 0,0013 mole), P.F. = 173°C, est obtenue (rendement 20 %).

***Exemple 9 :**

L'Exemple 4 est répété, avec remplacement des 3-méthyl-2-thiophènecarboxaldéhyde-(4′/5′-méthylpyridaz-3′-yl)hydrazones (1,75 g) par de la 2-thiophènecarboxaldéhyde-(5′-méthylpyridaz-3′-yl)hydrazone (1,70 g, 0,0078 mole) (préparée de la manière indiquée dans l'Exemple I-7). De la chloramine T (2,20 g, 0,0078 mole) est utilisée dans ce cas. Après chromatographie (éluant acétate d'éthyle), de la 7-méthyl-3-(thiène-2′-yl)-s-triazolo-[4,3-b]pyridazine (1,10 g, 0,0051 mole), P.F. = 198°C, est obtenue (rendement 65 %).

***Exemple I-1 :**

L'Exemple 6 est répété, avec remplacement :

– des 3-hydrazino-4/5-méthylpyridazines (1,25 g) par la 3-chloro-6-hydrazinopyridazine (5,0 g, 0,035 mole) (préparée de la manière indiquée dans l'Exemple I-8), et

– du chlorure de 2-fluorobenzoyle (1,59 g) par le chlorure de benzoyle (5,5 g, 0,039 mole). Après chromatographie (éluant heptane/acétate d'éthyle dans le rapport 30/70), de la 6-chloro-3-phényl-s-triazolo-[4,3-b]pyridazine (5,7 g, 0,025 mole), P.F. = 95°C, est obtenue (rendement = 71 %).

***Exemple I-2 :**

L'Exemple 6 est répété, avec remplacement :

– des 3-hydrazino-4/5-méthylpyridazines (1,25 g) par un mélange de 3-chloro-6-hydrazino-4/5-méthylpyridazines (0,75 g, 0,0047 mole) (préparées de la manière indiquée dans l'Exemple I-9), et

– du chlorure de 2-fluorobenzoyle (1,59 g) par le chlorure de benzoyle (0,66 g, 0,0047 mole).

Après chromatographie (éluant acétate d'éthyle), de la 6-chloro-8-méthyl-3-phényl-s-triazolo-[4,3-b]pyridazine (0,64 g, 0,0026 mole), P.F. = 184°C, et de la 6-chloro-7-méthyl-3-phényl-s-triazolo-[4,3-b]pyridazine (0,07 g, 0,0003 mole), P.F. = 170°C, sont obtenues (rendement 61 %).

***Exemple I-3 :**

Un mélange de 2-formyl-3-méthylthiophène (1,27 g, 0,01 mole) et de 3-hydrazino-4/5-méthylpyridazines (1,25 g, 0,01 mole) (préparées de la manière indiquée dans l'Exemple I-5) dans de l'éthanol (20 ml) contenant quelques gouttes d'acide chlorhydrique concentré est chauffé à la température d'ébullition pendant 2 heures. Puis le mélange réactionnel est concentré et lavé avec de l'éther diisopropylique. Le mélange de 3-méthyl-2-thiophènecarboxaldéhyde-(4'/5'-méthylpyridaz-3'-yl)hydrazones (2,07 g, 0,009 mole) obtenu est utilisé à l'état brut sans autre purification (rendement 89 %).

En procédant de manière similaire, on a préparé les composés suivants à partir des matières de départ substituées de la manière appropriée.

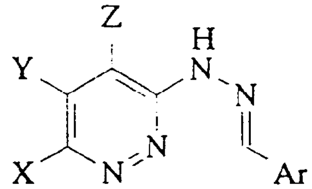

| Z | Y | X | Ar |
|---|---|---|---|
| Me | Me | H | Phényle |
| Me/H | H/Me | H | 2-Méthylphényle |
| Me/H | H/Me | H | 4-Méthylphényle |
| Me/H | H/Me | H | 3-Méthylphényle |
| Me/H | H/Me | H | 1-Méthylpyrrole-2-yle |
| Me/H | H/Me | H | 4-Isopropylphényle |
| Me/H | H/Me | H | 4-Phénoxyphényle |
| Me/H | H/Me | H | 4-Ethylphényle |
| Me/H | H/Me | H | 2-Bromophényle |
| Me/H | H/Me | H | 3-Phénoxyphényle |
| Me/H | H/Me | H | 3-(4'-Chlorophénoxy)phényle |
| Me/H | H/Me | H | 3-Fluoro-2-méthylphényle |
| Me/H | H/Me | H | 4-Bromophényle |

Par remplacement du 2-formylthiophène par l'acétal éthylénique du 3-formyl-2-méthylthiothiophène, les conditions réactionnelles précitées sont utilisées.

Après 4 heures de reflux, le mélange réactionnel est refroidi, ce qui provoque la précipitation d'une substance solide. Après filtration et chromatographie de la substance solide (éluant consistant un un mélange pétrole 60:80/éther/acétonitrile/acétate d'éthyle), de la 2-méthylthio-3-thiophène-(5'-méthylpyridaz-3'-yl)hydrazone est obtenue, P.F. 198°C. Après concentration du filtrat et chromatographie (éluant consistant en un mélange pétrole 60:80/éther/acétonitrile/acétate d'éthyle), de la 2-méthylthio-3-thiophène-(4'-méthylpyridaz-3'-yl)hydrazone, P.F. 168°C, est obtenue (rendement 60 %).

***Exemple I-4 :**

L'Exemple I-3 est répété, avec remplacement du 2-formyl-3-méthylthiophène par le 3-formylthiophène (1,35 g, 0,0121 mole). Le mélange de 3-hydrazino-4/5-méthylpyridazines (1,50 g, 0,0121 mole) est utilisé dans ce cas. Un mélange de 3-thiophènecarboxaldéhyde-(4'/5'-méthylpyridaz-3'-yl)hydrazones (2,44 g, 0,0112 mole) est obtenu et utilisé sans autre purification (rendement 93 %).

***Exemple I-5 :**

Un mélange de 3-chloro-6-hydrazino-4/5-méthylpyridazines (9,65 g, 0,061 mole) (préparées de la manière indiquée dans l'Exemple I-9), d'un catalyseur à 10 % de palladium sur du carbone (1,8 g) et d'une solution à 98 % d'hydrate d'hydrazine (40 ml) dans du méthanol (300 ml) est chauffé à la température de reflux du solvant pendant un temps d'une heure trente minutes. Le mélange chaud est filtré sur du Hyflo-supercel et concentré. On obtient un mélange de 3-hydrazino-4/5-méthylpyridazines (4,8 g, 0,039 mole), qui est cristallisé dans le toluène et utilisé sans autre purification (rendement 64 %).

De la 3-hydrazino-4,5-diméthylpyridazine a été préparée de manière similaire à partir de 3-chloro-6-hydrazino-4,5-diméthylpyridazine.

***Exemple I-6 :**

L'Exemple I-3 est répété, avec remplacement :
– du 2-formyl-3-méthylthiophène (1,27 g) par le 2,4-diméthylbenzaldéhyde (1,62 g, 0,0121 mole), et
– des 3-hydrazino-4/5-méthylpyridazines (1,25 g) par le même mélange (1,5 g, 0,0121 mole).

Un mélange de 2,4-diméthylbenzaldéhyde-(4'/5'-méthylpyridaz-3'-yl)hydrazones (2,35 g, 0,0098 mole) obtenu est utilisé à l'état brut sans autre purification (rendement 81 %).

***Exemple I-7 :**

L'Exemple I-3 est répété, avec remplacement :
– du 2-formyl-3-méthylthiophène (1,27 g) par le 2-formylthiophène (1,9 g, 0,017 mole) et
– des 3-hydrazino-4/5-méthylpyridazines (1,25 g) par le même mélange (2,1 g, 0,017 mole).

Après 1 heure à la température de reflux du solvant puis un refroidissement, de la 2-thiophènecarboxaldéhyde-(5'-méthylpyridaz-3'-yl)hydrazone (1,8 g, 0,0082 mole) est obtenue par filtration. L'évaporation du filtrat rend possible la séparation, après lavage avec de l'éther diisopropylique, de 2-thiophènecarboxaldéhyde-(4'-méthylpyridaz-3'-yl)hydrazone (1,4 g, 0,0064 mole). Ces deux intermédiaires sont utilisés à l'état brut sans autre purification (rendement 86 %).

***Exemple I-8 :**

De la 3-chloro-6-hydrazinopyridazine est préparée suivant un mode opératoire décrit dans la littérature :
Carlo FARINA, Riccardo MONGUZZI et Marino PINZA, Organic Preparations and Procedures Int., 21 (1), 125-128 (1989).

***Exemple I-9 :**

Des 3-chloro-6-hydrazino-4/5-méthylpyridazines sont obtenues suivant le procédé de :
Soeren LINHOLTER et Regitze ROSENOERN, Acta Chem. Scand., 16, 2389-2394 (1962).

***Exemple I-10**

De la 3-chloro-6-hydrazino-4,5-diméthylpyridazine est obtenue suivant le procédé de :
M. JAPELJ, B. STANOVNIK et M. TISLER, Monatsch. Chem., 100, 671, (1969).

***Exemple I-11 :**

Les chlorures de benzoyle utilisés pour la préparation des composés 19 et 23 ont été préparés par chauffage des acides benzoïques, substitués de la manière appropriée, au reflux avec du chlorure de thionyle pendant un temps de 3 heures. Le chlorure de thionyle en excès est éliminé par évaporation et les chlorures de benzoyle sont utilisés directement sans autre purification.

***Exemple I-12**

Un mélange de 3-formylthiophène (50,5 g, 0,45 mole), d'éthylèneglycol (25,2 ml, 0,45 mole) dans du toluène contenant une quantité catalytique d'acide paratoluènesulfonique est chauffé au reflux pendant 3 heures 30 minutes. Aprés évaporation du solvant, l'huile résultante est distillée et de l'acétal éthylénique de 3-formylthiophène, E.b. 72-74°C/1 mm de Hg est obtenu (rendement 55 %).

***Exemple I-13 :**

Du n-butyl-lithium (2,5 M dans l'hexane, 36 ml) est ajouté sous atmosphère inerte à une solution refroidie, sous agitation, d'acétal éthylénique de 3-formylthiophène (13,0 g, 0,083 mole) dans de l'éther, la température étant maintenue à une valeur inférieure à -70°C. On agite le mélange à -70°C pendant 1 heure, puis on le laisse se réchauffer à température ambiante pendant un temps supplémentaire de 30 minutes.

Le mélange est refroidi à nouveau a une température d'approximativement -78°C et une solution de disulfure de méthyle (9,0 ml, 0,1 mole) dans l'éther est ajoutée en un temps de 5 minutes. On agite le mélange à -78°C pendant 40 minutes, puis on le laisse se réchauffer à température ambiante pendant 16 heures.

Le mélange est versé dans un mélange de glace et d'eau et soumis à une extraction avec du chlorure de méthylène. Les phases organiques réunies sont déshydratées (sulfate de sodium anhydre), filtrées et évaporées, ce qui donne une huile jaune limpide. Après distillation, de l'acétal éthylénique de 3-formyl-2-méthylthiothiophène E.b. 130-132°C/1-4 mm de Hg, est obtenu (rendement 77 %).

Conformément à une caractéristique de la présente invention, il est proposé un procédé pour lutter contre la croissance de mauvaises herbes (c'est-à-dire de végétation indésirable) dans un milieu, qui consiste à appliquer à ce milieu une quantité, à effet herbicide, d'au moins un dérivé de triazolopyridazine de formule générale (I) ou d'un de ses sels acceptables en agriculture. A cette fin, les dérivés de triazolopyridazine sont utilisés habituellement sous forme de compositions herbicides (c'est-à-dire en association avec des diluants ou supports compatibles et/ou agents tensio-actifs pouvant être utilisés dans des compositions herbicides), par exemple de la manière décrite ci-après.

La présente invention concerne également un procédé pour lutter contre des mauvaises herbes, (en particulier dans des zones de cultures de plantes dicotylédones ou monocotylédones), qui consiste à appliquer à la zone qui doit être débarrassée des mauvaises herbes et/ou aux plantes qui doivent être détruites une quantité efficace d'un composé conforme à la présente invention, en particulier d'un composé de formule (I), les plantes elles-mêmes (ou mauvaises herbes) qui doivent être détruites ou dont la croissance doit être inhibée pouvant être du type monocotylédone ou dicotylédone.

Les produits et les compositions conformes à la présente invention sont aisément appliqués à la végétation et, en particulier, aux mauvaises herbes à éliminer lorsque ces dernières possèdent un feuillage vert (application en post-levée).

Les composés de formule générale (I) présentent une activité herbicide contre des mauvaises herbes dicotylédones (c'est-à-dire à feuilles larges) et monocotylédones (par exemple des graminées) par application en pré- et/ou post-levée.

L'expression "application en pré-levée" désigne l'application à la terre dans laquelle se trouvent les semences ou jeunes pousses de mauvaises herbes avant apparition des mauvaises herbes au-dessus de la surface du sol. L'expression "application en post-levée" désigne l'application aux parties aériennes ou à découvert des mauvaises herbes qui ont émergé au-dessus de la surface du sol. Par exemple, les composés de formule générale (I) peuvent être utilisés pour lutter contre la croissance :

de mauvaises herbes à feuilles larges, par exemple Abutilon theophrasti, Amaranthus retroflexus, Bidens pilosa, Chenopodium album, Chrysanthemum segetum, Galium aparine, Ipomoea spp., par exemple Ipomoea purpurea, Polygonum convolvulus, Sesbania exaltata, Sinapis arvensis, Sinapsis alba, Solanum nigrum, Stelloria media et Xanthium strumarium, et

de mauvaises herbes de la catégorie des graminées, par exemple Alopecurus myosuroides, Avena fatua, Digitaria sanguinalis, Echinochloa crus-galli, Eleusine indica et Setaria spp., par exemple Setaria faberii ou Setaria viridis, et

de souchets, par exemple Cyperus esculentus.

Les quantités de composés de formule générale (I) appliquées varient suivant la nature des mauvaises herbes, les compositions utilisées, le temps d'application, les conditions climatiques et édaphiques et (lorsque ces composés sont utilisés pour lutter contre la croissance de mauvaises herbes dans des zones de cultures) la nature des cultures. Lorsque les composés sont appliqués à une zone de culture, le taux d'application doit être suffisant pour lutter contre la croissance des mauvaises herbes sans provoquer un endommagement permanent notable de la culture. En général, ces facteurs étant pris en considération, des taux d'application de 0,01 kg à 20 kg de matière active par hectare, avantageusement de 0,5 kg à 8,0 kg de matière active par hectare, de préférence de 0,4 à 4 kg/ha, donnent de bons résultats. Cependant, il doit être entendu que des taux d'application supérieurs ou inférieurs peuvent être utilisés suivant le problème particulier de destruction de mauvaises herbes rencontré.

Les composés de formule générale (I) peuvent être utilisés pour lutter sélectivement contre la croissance

de mauvaises herbes, par exemple pour lutter contre la croissance des espèces décrites précédemment, par application en pré- ou post-levée de manière directionnelle ou non directionnelle, par exemple par pulvérisation directionnelle ou non directionnelle, à un milieu infesté par des mauvaises herbes qui consiste en une zone utilisée, ou destinée à être utilisée, pour des cultures, par exemple de céréales, telles que le blé, l'orge, l'avoine, le maïs et le riz, le soja, la féverole commune et les haricots nains, les pois, la luzerne, le cotonnier, les arachides, le lin, les oignons, les carottes, les choux, le colza, le tournesol, la betterave sucrière et des herbages permanents ou obtenus par semis, avant ou après plantation de la culture ou bien avant ou après levée de la culture. Pour la lutte sélective contre des mauvaises herbes dans un milieu infesté par ces mauvaises herbes qui consiste en une zone utilisée, ou destinée à être utilisée, pour des cultures, par exemple les cultures mentionnées précédemment, des taux d'application de 0,01 kg à 8,0 kg, et de préférence de 0,01 kg à 4,0 kg, de matière active par hectare conviennent particulièrement.

Les composés de formule générale (I) peuvent également être utilisés pour lutter contre la croissance de mauvaises herbes, notamment des mauvaises herbes précitées, par application en pré- ou post-levée dans des vergers de plantation et d'autres zones de croissance d'arbres, par exemple des forêts, des bois et des parcs, ainsi que des plantations, par exemple des plantations de canne à sucre, de palmiers à huile et d'arbres à caoutchouc. A cette fin, ils peuvent être appliqués de manière directionnelle ou non directionnelle (par exemple par pulvérisation directionnelle ou non directionnelle) aux mauvaises herbes ou bien au sol dans lequel ces mauvaises herbes doivent apparaître, avant ou après plantation des arbres ou établissement de plantations, à des taux d'application de 0,25 kg à 10,0 kg par hectare.

Les composés de formule générale (I) peuvent également être utilisés pour lutter contre la croissance de mauvaises herbes, notamment des mauvaises herbes précitées, dans des milieux qui ne sont pas des zones de cultures mais dans lesquelles il est néanmoins désiré de lutter contre des mauvaises herbes.

Des exemples de ces zones non destinées aux cultures comprennent des terrains d'aviation, des cités industrielles, des voies de chemin de fer, les bas-côtés des routes, les bords des rivières, des voies d'irrigation et d'autres voies d'eau, des terrains broussailleux et des terrains en friche ou non cultivés, en particulier dans lesquels il est désiré de lutter contre la croissance de mauvaises herbes afin de réduire les risques d'incendie. Lorsqu'ils sont utilisés à de telles fins, dans lesquelles un effet herbicide total est fréquemment désiré, les composés actifs sont habituellement appliqués à des doses supérieures à celles utilisées dans les zones de culture décrites précédemment. La dose précise dépend de la nature de la végétation traitée et de l'effet recherché.

Une application en pré- ou post-levée, et de préférence une application en pré-levée, de manière directionnelle ou non directionnelle (par exemple par pulvérisation directionnelle ou non directionnelle) à des taux d'application de 1,0 kg à 20,0 kg, et de préférence de 5,0 à 10,0 kg, de matière active par hectare conviennent particulièrement à cette fin.

Lorsqu'ils sont utilisés pour lutter contre la croissance de mauvaises herbes par application en prélevée, les composés de formule générale (I) peuvent être incorporés à la terre dans laquelle la levée des mauvaises herbes est prévue. On notera que, lorsque les composés de formule générale (I) sont utilisés pour lutter contre la croissance de mauvaises herbes par application en post-levée, c'est-à-dire par application aux parties aériennes ou à découvert de mauvaises herbes ayant levé, les composés de formule générale (I) viennent en outre habituellement en contact avec le sol et peuvent alors permettre de lutter en pré-levée contre des mauvaises herbes présentant une germination ultérieure dans le sol.

Des composés représentatifs de formule générale (I) ont été utilisés dans des applications ou utilisations herbicides conformément aux modes opératoires suivants.

Les exemples ci-dessous, présentés à titre non limitatif, illustrent l'utilisation des produits conformes à la présente invention et leur application à la lutte contre des mauvaises herbes.

Les abréviations suivantes sont utilisées dans ces exemples :

| Abréviations | Nom français de l'espèce de mauvaise herbe | Nom latin |
|---|---|---|
| AVE | Folle avoine | Avena fatua |
| ECH | Panic pied-de-coq | Echinochloa crus-galli |
| DIG | Digitaire sanguine | Digitaria sanguinalis |
| SIN | Moutarde | Sinapis alba |
| ALO | Vulpin des champs | Alopecurus myosuroides |
| ABU | Abutilon | Abutilon theophrasti |
| SOL | Morelle noire | Solanum nigrum |
| STE | Mouron blanc | Stellaria media |
| SES | Sesbania | Sesbania exaltata |
| CHY | Chrysanthème des moissons | Chrysanthemum segetum |

| Abréviations | Nom français | Nom latin de l'espèce cultivée |
|---|---|---|
| TRZ | Blé | Triticum aestivum |
| ZEA | Maïs | Zea mays |
| ORY | Riz | Oryza sativa |
| GLX | Soja | Glycine maximum |
| HEL | Tournesol | Helianthus annuus |
| BRS | Colza | Brassica napus |

**Exemple U1 :**

Traitement herbicide en pré-levée d'espèces de plantes

Un certain nombre de semences, déterminé en fonction de l'espèce de plante et de la grosseur des semences, est semé dans des pots de 7 x 7 x 8 cm remplis de terre agricole légère.

Les pots sont traités par pulvérisation d'un mélange herbicide en une quantité correspondant à une dose appliquée, en volume, de 500 l/ha et contenant la matière active à la concentration désirée.

Le traitement avec le mélange est donc effectué sur les semences non recouvertes de terre (le terme "mélange" est utilisé de manière générale pour désigner les compositions diluées avec de l'eau, telles qu'elles sont appliquées aux plantes).

Le mélange utilisé pour le traitement est une solution ou suspension de la matière active dans un mélange

acétone/eau dans le rapport 50/50, en présence de 0,05 % en poids de Cemulsol NP 10 (un agent tensio-actif consistant en un produit de polycondensation de l'oxyde d'éthylène avec un alkylphénol, en particulier un produit de polycondensation de l'oxyde d'éthylène avec le nonylphénol) et 0,04 % en poids de Tween 20 (agent tensio-actif consistant en un ester oléique d'un produit de polycondensation de l'oxyde d'éthylène avec un dérivé de sorbitol).

Dans le cas d'une suspension, cette dernière est obtenue par mélange et broyage des ingrédients dans un appareil de micronisation de manière à parvenir à un diamètre moyen de particules inférieur à 40 micromètres.

Après traitement, les semences sont recouvertes d'une couche de terre d'environ 3 mm d'épaisseur.

Puis les pots sont placés dans des réservoirs destinés à recevoir l'eau pour l'arrosage, dans des conditions de sub-irrigation, et sont maintenus pendant 24 jours à température ambiante à une humidité relative de 60 %.

Les composés utilisés pour le traitement des plantes ont été testés sur les espèces de mauvaises herbes suivantes : AVE, ECH, ALO, DIG, SIN, ABU, SOL, STE et CHY, et sur les espèces de plantes cultivées suivantes : ORY, BRS, GLX, HEL et ZEA.

Au bout de 24 jours, une évaluation visuelle de l'endommagement des plantes cultivées a été effectuée, les résultats étant exprimés en pourcentage de réduction de croissance ou d'endommagement des plantes cultivées ou des mauvaises herbes, par comparaison aux plantes dans les pots témoins.

**Exemple U2 :**

Traitement herbicide en post-levée d'espèces de plantes

Un certain nombre de semences, déterminé en fonction de l'espèce de plante et de la grosseur des semences, est semé dans des pots de 7 x 7 x 8 cm remplis de terre agricole légère. Puis les semences sont recouvertes d'une couche de terre d'environ 3 mm d'épaisseur et les semences sont laissées en germination jusqu'à ce qu'elles engendrent une plantule au stade approprié. Le stade de traitement pour les graminées est le stade "de formation de la deuxième feuille". Le stade de traitement pour les plantes dicotylédones est le stade "d'étalement des cotylédons, et d'étalement de la première vraie feuille".

Puis les pots sont traités par pulvérisation d'un mélange herbicide en une quantité correspondant à une dose appliquée, en volume, de 500 l/ha et contenant la matière active à la concentration désirée. Le mélange utilisé pour le traitement est préparé de la manière indiquée dans l'Exemple U1. Après traitement, les semences sont recouvertes d'une couche de terre d'environ 3 mm d'épaisseur.

Puis les pots sont placés dans des réservoirs destinés à recevoir l'eau pour l'arrosage, dans des conditions de sub-irrigation, et sont maintenus pendant 24 heures à température ambiante à une humidité relative de 60 %. L'évaluation de la phytotoxicité a été effectuée de la manière indiquée dans l'Exemple U1. Les composés utilisés pour le traitement des plantes ont été testés sur les espèces de mauvaises herbes suivantes : AVE, ECH, ALO, DIG, SIN, ABU, SOL, STE et CHY, et sur les espèces de plantes cultivées suivantes : ORY, TRZ et ZEA.

**Exemple U3 :**

Traitement herbicide en pré-levée d'espèces de plantes

Des quantités appropriées des composés utilisés pour le traitement des plantes ont été dissoutes dans de l'acétone pour obtenir des solutions permettant des taux d'application allant jusqu'à 4000 g de composé d'essai par hectare (g/ha). Ces solutions ont été appliquées au moyen d'un pulvérisateur d'herbicide usuel de laboratoire délivrant l'équivalent de 290 litres de liquide de pulvérisation par hectare. Les semences ont été semées dans des pots en matière plastique de section carrée de 70 mm de côté et de 75 mm de profondeur dans de la terre non stérile. Les quantités de semences par pot étaient les suivantes :

| Espèces de mauvaises herbes | Nombre approximatif de semences/pot |
|---|---|
| 1) Mauvaises herbes à feuilles larges | |
| Abutilon theophrasti | 10 |
| Amaranthus retroflexus | 20 |
| Galium aparine | 10 |
| Ipomoea purpurea | 10 |
| Sinapis arvensis | 15 |
| Xanthium strumarium | 2 |
| 2) Mauvaises herbes de la catégorie des graminées | |
| Alopecurus myosuroides | 15 |
| Avena fatua | 10 |
| Echinochloa crus-galli | 15 |
| Setaria viridis | 20 |
| 3) Souchets | |
| Cyperus esculentus | 3 |
| Plantes cultivées | |
| 1) A feuilles larges | |
| Cotonnier | 3 |
| Soja. | 3 |
| 2) Graminées | |
| Maïs | 2 |
| Riz | 6 |
| Blé | 6 |

Les composés de la présente invention ont été appliqués à la surface du sol contenant les semences. Un seul pot de chaque plante cultivée et de chaque mauvaise herbe a été soumis à chaque traitement, accompagné de témoins n'ayant pas subi de pulvérisations et de témoins ayant été soumis à une pulvérisation avec de l'acétone seule.

Après traitement, les pots ont été placés sur une natte à effet capillaire maintenue dans une serre et ont été arrosés par le dessus. L'évaluation visuelle de l'endommagement des plantes cultivées a été effectuée 20 à 24 jours après pulvérisation. Les résultats ont été exprimés en pourcentage de réduction de croissance ou d'endommagement des plantes cultivées ou des semences, comparativement aux plantes présentes dans les pots témoins.

**Exemple U4 :**

Traitement herbicide en pré-levée d'espèces de plantes

Les mauvaises herbes et les plantes cultivées ont été semées directement dans du compost de mise en pot John Innes dans des pots de 75 mm de profondeur et de 70 mm de côté, à l'exception de la plante du genre Amaranthus qui a été repiquée par prélèvement au stade plant de semis et transfert dans les pots une semaine

avant pulvérisation. Puis les plantes ont été cultivées dans la serre jusqu'à ce qu'elles soient prêtes à être soumises à une pulvérisation des composés utilisés pour le traitement des plantes. Le nombre de plants par pot était le suivant :

Mauvaises herbes

1) Mauvaises herbes à feuilles larges

| Espèce de mauvaise herbe | Nombre de plants par pot | Stade de croissance |
|---|---|---|
| Abutilon theophrasti | 3 | 1-2 feuilles |
| Amaranthus retroflexus | 4 | 1-2 feuilles |
| Galium aparine | 3 | 1er verticille |
| Ipomoea purpurea | 3 | 1-2 feuilles |
| Sinapis arvensis | 4 | 2 feuilles |
| Xanthium strumarium | 1 | 2-3 feuilles |

2) Mauvaises herbes de la catégorie des graminées

| Espèce de mauvaise herbe | Nombre de plants par pot | Stade de croissance |
|---|---|---|
| Alopecurus myosuroides | 8-12 | 1-2 feuilles |
| Avena fatua | 12-18 | 1-2 feuilles |
| Echinochloa crus-galli | 4 | 2-3 feuilles |
| Setaria viridis | 15-25 | 1-2 feuilles |

3) Souchets

| Espèce de mauvaise herbe | Nombre de plants par pot | Stade de croissance |
|---|---|---|
| Cyperus esculentus | 3 | 3 feuilles |

Plantes cultivées

1) A feuilles larges

| | Nombre de plants par pot | Stade de croissance |
|---|---|---|
| Cotonnier | 2 | 1 feuille |
| Soja | 2 | 2 feuilles |

2) Graminées

| | Nombre de plants par pot | Stade de croissance |
|---|---|---|
| Maïs | 2 | 2-3 feuilles |
| Riz | 4 | 2-3 feuilles |
| Blé | 5 | 2-3 feuilles |

Le mélange utilisé pour le traitement a été préparé de la manière indiquée dans l'Exemple U1 et l'évaluation de la phytotoxicité a été effectuée de la manière indiquée dans l'Exemple U1. Un seul pot de chaque plante cultivée et de chaque espèce de mauvaise herbe a été soumis à chaque traitement, avec des témoins n'ayant pas subi de pulvérisation et des témoins ayant été soumis à une pulvérisation avec de l'acétone seule.

Après traitement, les pots ont été placés sur une natte à effet capillaire dans une serre et ont été arrosés une fois par le dessus après un temps de 24 heures, puis ont été arrosés par sub-irrigation contrôlée. L'évaluation visuelle de l'endommagement des plantes cultivées et de la destruction des mauvaises herbes a été effectuée 20 à 24 jours après pulvérisation. Les résultats ont été exprimés en pourcentage de réduction de croissance ou d'endommagement des plantes cultivées ou des mauvaises herbes, par comparaison aux plantes dans les pots témoins.

Des composés représentatifs de la présente invention, lorsqu'ils ont été utilisés à un taux égal ou inférieur à 4 kg/ha, ont présenté un degré excellent d'activité herbicide sur les mauvaises herbes utilisées dans les expériences précédentes, conjointement avec un excellent degré de tolérance vis-à-vis des cultures.

Lorsqu'ils ont été appliqués à 4 kg/ha conformément au procédé décrit dans l'Exemple U1, les composés 1, 2, 3, 4a, 5a, 6a, 7a, 8, 10, 11, 12, 13 et 14 ont provoqué au moins 90 % de réduction de la croissance d'une ou plusieurs espèces de mauvaises herbes.

Lorsqu'ils ont été appliqués à 4 kg/ha conformément au procédé décrit dans l'Exemple U2, les composés 1, 2, 3, 4a, 5a, 6a, 7a, 8, 10, 11, 12, 13 et 14 ont engendré au moins 90 % de réduction de la croissance d'une ou plusieurs espèces de mauvaises herbes.

Lorsqu'ils ont été appliqués à 2 kg/ha conformément au procédé décrit dans l'Exemple U3, les composés 15, 16a, 16b, 17, 18, 19, 20a, 21, 22a, 23, 24, 25b et 27 ont engendré au moins 90 % de réduction de la croissance d'une ou plusieurs espèces de mauvaises herbes, avec une sélectivité vis-à-vis d'au moins une espèce de plante cultivée.

Lorsqu'ils ont été appliqués à 2 kg/ha conformément au procédé décrit dans l'Exemple U4, les composés 18, 19, 20a, 20b 22b, 24, 25a, 26 ont engendré au moins 90 % de réduction de la croissance d'une ou plusieurs espèces de mauvaises herbes, avec une sélectivité vis-à-vis d'au moins une espèce de plante cultivée.

Pour leur utilisation pratique, les composés de formule générale (I) sont rarement utilisés seuls. Le plus souvent, ces composés font partie de compositions. Ces compositions, qui peuvent être utilisées comme agents herbicides, contiennent comme matière active un composé de formule générale (I) répondant à la définition précitée ou un de ses sels acceptables en agriculture, en mélange avec au moins un support solide ou liquide acceptable en agriculture et, facultativement, au moins un agent tensioactif également acceptable en agriculture. Dans la présente description, il est entendu que l'expression "agent tensioactif" désigne un agent

connu sous le nom de "surfactant", terme qui désigne principalement des agents tensio-actifs, mouillants ou dispersants.

Ces compositions font également partie de la présente invention. Elles peuvent également contenir toutes sortes d'autres ingrédients tels que, par exemple, des colloïdes protecteurs, des adhésifs, des épaississants, des agents thixotropes, des agents accroissant la capacité de pénétration, des stabilisants, des séquestrants, etc. Plus généralement, les composés utilisés dans la présente invention peuvent être associés à tous les additifs solides ou liquides correspondant aux procédés usuels de préparation de formulations.

En général, les compositions conformes à la présente invention contiennent habituellement environ 0,05 à 95 % (en poids) d'une composition conforme à la présente invention, un ou plusieurs supports solides ou liquides et, facultativement, un ou plusieurs agents tensio-actifs.

Le terme "support" désigne dans la présente description une matière organique ou inorganique, naturelle ou synthétique, à laquelle le composé est mélangé afin de faciliter l'application de ce composé à la plante, aux semences ou au sol. Ce support est donc généralement inerte et doit être acceptable en agriculture, en particulier sur la plante traitée.

Le support peut être de n'importe quel type usuel. En particulier, il peut être solide (argiles, silicates naturels ou synthétiques, silice, résines, cires, engrais solides, etc.), ou liquide (eau, alcools, en particulier le butanol, etc.).

L'agent tensio-actif ou surfactant peut également être de n'importe quel type usuel. Il peut s'agir d'un agent émulsionnant, dispersant ou mouillant du type ionique ou non ionique ou bien d'un mélange de tels agents tensio-actifs. On peut citer, par exemple, des sels d'acides polyacryliques, des sels d'acide lignosulfonique, des acides phénolsulfoniques ou naphtalènesulfoniques, des produits de polycondensation de l'oxyde d'éthylène avec des alcools gras, avec des acides gras ou avec des amines grasses, des phénols substitués (en particulier des alkylphénols ou des arylphénols), des sels d'esters d'acide sulfosuccinique, des dérivés de taurine (en particulier des taurates d'alkyle), des esters phosphoriques de produits de polycondensation de l'oxyde d'éthylène avec des alcools ou des phénols, des esters d'acides gras et de polyols, des dérivés des composés précédents avec des groupes sulfate, sulfonate et phosphate.

La présence d'au moins un agent tensio-actif est généralement essentielle lorsque le composé inerte et/ou le support ne sont pas solubles dans l'eau et lorsque le véhicule d'application est l'eau.

Ainsi, les compositions destinées à être utilisées en agriculture conformément à la présente invention peuvent contenir les matières actives conformes à la présente invention dans de très larges limites, de 0,05 % à 95 % (en poids). Leur teneur en agent tensioactif est avantageusement comprise dans l'intervalle de 0,1 % à 50 % en poids.

Dans le cas des compositions aptes à l'entreposage et au transport, ces compositions contiennent avantageusement 0,5 à 95 % (en poids) de substance active, les compositions telles qu'elles sont appliquées aux plantes étant en général notablement plus diluées que les compositions aptes à l'entreposage et au transport, qui sont plus concentrées.

Les compositions conformes à la présente invention sont elles-mêmes sous des formes solides ou liquides relativement diverses.

Comme formes de compositions solides, on peut mentionner des poudres pour poudrage (avec une teneur en composé qui peut atteindre 100 %), des poudres mouillables et des granules, en particulier ceux obtenus par extrusion, compactage et imprégnation d'un support granulé ou par granulation d'une poudre (la teneur en composé de ces granules allant de 0,5 à 80 % pour ces derniers cas).

Les poudres mouillables (ou poudres pour pulvérisation) sont habituellement préparées de manière à contenir 20 à 95 % de matière active et elles contiennent habituellement, en plus du support solide, 0 à 30 % d'un agent mouillant, 3 à 20 % d'un agent dispersant et, lorsque cela est nécessaire, 0 à 10 % d'un ou plusieurs stabilisants et/ou autres additifs tels que des agents accroissant la capacité de pénétration, des adhésifs ou des agents inhibant la floculation, des colorants, etc.

Pour obtenir les poudres pour pulvérisation ou poudres mouillables, les matières actives sont mélangées intimement dans des mélangeurs convenables aux substances additionnelles et sont broyées dans des moulins ou autres broyeurs convenables. On obtient ainsi des poudres pour pulvérisation dont la mouillabilité et la capacité de mise en suspension sont avantageuses ; ces poudres peuvent être mises en suspension dans l'eau à n'importe quelle concentration désirée et ces suspensions peuvent être utilisées de manière très avantageuse, en particulier pour l'application aux feuilles des plantes.

Des pâtes peuvent être préparées à la place des poudres mouillables. Les conditions et les détails de la préparation et de l'utilisation de ces pâtes sont similaires à celles des poudres mouillables ou poudres pour pulvérisation.

A titre d'exemple, différentes compositions de poudres mouillables (ou de poudres pour pulvérisation) sont présentées :

**Exemple F 1 :**

| | | | |
|---|---|---|---|
| * | Matière active (composé N° 1) | 50 | % |
| * | Produit de condensation oxyde d'éthylène/alcool gras (agent mouillant) | 2,5 | % |
| * | Produit de condensation oxyde d'éthylène/phényl-éthylphénol (agent dispersant) | 5 | % |
| * | Chaux (support inerte) | 42,5 | % |

**Exemple F 2 :**

| | | | |
|---|---|---|---|
| * | Matière active (composé N° 1) | 10 | % |
| * | 8 à 10 motifs oxyde d'éthylène/alcool synthétique à fonction oxo, en $C_{13}$, du type ramifié (agent mouillant) | 0,75 | % |
| * | Lignosulfonate de calcium neutre (agent dispersant) | 12 | % |
| * | Carbonate de calcium (support inerte) q.s. | 100 | % |

**Exemple F 3 :** cette poudre mouillable contient les mêmes ingrédients que dans l'exemple précédent, en les proportions ci-dessous :

| | | | |
|---|---|---|---|
| * | Matière active | 75 | % |
| * | Agent mouillant | 1,50 | % |
| * | Agent dispersant | 8 | % |
| * | Carbonate de calcium (charge inerte) q.s. | 100 | % |

**Exemple F 4 :**

| | | | |
|---|---|---|---|
| * | Matière active (composé N° 1) | 90 | % |
| * | Produit de condensation oxyde d'éthylène/alcool gras (agent mouillant) | 4 | % |
| * | Produit de condensation oxyde d'éthylène/phényléthylphénol (agent dispersant) | 6 | % |

**Exemple F 5 :**

| | | | |
|---|---|---|---|
| * | Matière active (composé N° 1) | 50 | % |
| * | Mélange d'agents tensio-actifs anioniques et non ioniques (agent mouillant) | 2,5 | % |
| * | Lignosulfonate de sodium (agent dispersant) | 5 | % |
| * | Argile du type kaolin (support inerte) | 42,5 | % |

Les composés conformes à la présente invention peuvent être formulés en granules dispersables dans l'eau, qui entrent également dans le cadre de la présente invention.

Ces granules dispersables, ayant généralement une densité apparente d'environ 0,3 à 0,6, possèdent un diamètre de particules compris généralement dans l'intervalle d'environ 0,15 à 2 mm et de préférence de 0,3 à 1,5 mm.

La teneur en matière active de ces granules est généralement comprise dans l'intervalle d'environ 1 % à 90 %, et de préférence de 25 % à 90 %.

Le reste du granule est constitué essentiellement d'une charge solide et, facultativement, d'adjuvants tensio-actifs qui confèrent au granule des propriétés de dispersibilité dans l'eau. Ces granules peuvent être essentiellement de deux types distincts suivant le fait que la charge choisie est soluble ou insoluble dans l'eau. Lorsque la charge est hydrosoluble, cette charge peut être inorganique ou, de préférence, organique. Des résultats excellents ont été obtenus avec l'urée. Dans le cas d'une charge insoluble, cette dernière est de préférence inorganique, un exemple d'une telle charge étant le kaolin ou la bentonite. Dans ce cas, elle est accompagnée avantageusement d'agents tensio-actifs (en une quantité de 2 à 20 % en poids du granule) dont plus de la moitié, par exemple, consiste en au moins un agent dispersant essentiellement anionique, tel qu'un polynaphtalènesulfonate de métal alcalin ou de métal alcalino-terreux ou un lignosulfonate de métal alcalin ou de métal alcalino-terreux, le reste consistant en agents mouillants non ioniques ou anioniques tels qu'un alkylnaphtalènesulfonate de métal alcalin ou de métal alcalino-terreux.

En outre, bien que cela ne soit pas essentiel, d'autres adjuvants tels que des agents antimousse peuvent être ajoutés.

Le granule conforme à la présente invention peut être préparé par mélange des ingrédients nécessaires, puis granulation suivant plusieurs procédés en eux-mêmes connus (tambour de confiserie, lit fluide, atomiseur, extrusion, etc.). La mise en oeuvre du procédé est généralement achevée par broyage, puis par tamisage au diamètre de particules choisi, dans les limites précitées.

De préférence, le granule est obtenu par extrusion, les modes opératoires mentionnés dans les Exemples ci-dessous étant suivis.

**Exemple F 6 :** Granules dispersables

La matière active (composé N° 1) (90 % en poids) et de l'urée en perles (10 %) sont mélangées dans un

mélangeur. Puis le mélange est broyé dans un broyeur à cylindre denté. On obtient une poudre qui est humidifiée avec de l'eau (environ 8 % en poids). La poudre humide est extrudée dans une extrudeuse à cylindre perforé. Cela donne un granule qui est séché, puis broyé et tamisé de manière à retenir seulement les granules ayant des diamètres compris dans l'intervalle de 0,15 à 2 mm.

**Exemple F 7 :** Granules dispersables

Les constituants suivants sont mélangés dans un mélangeur :

| | |
|---|---|
| * Matière active (composé N° 1) | 75 % |
| * Agent mouillant (alkylnaphtalène-sulfonate de sodium) | 2 % |
| * Agent dispersant (polynaphtalène-sulfonate de sodium) | 8 % |
| * Charge inerte insoluble dans l'eau (kaolin) | 15 % |

Ce mélange est granulé en lit fluide en présence d'eau, puis est séché, broyé et tamisé de manière à obtenir des granules ayant des diamètres compris dans l'intervalle de 0,15 à 0,80 mm.

Ces granules peuvent être utilisés seuls, en solution ou dispersés dans l'eau de manière à obtenir la dose désirée. Ils peuvent être utilisés également pour la préparation de mélanges avec d'autres matières actives, en particulier des herbicides, ces derniers étant sous forme de poudres ou granules mouillables ou bien de suspensions aqueuses.

Les composés de formule (I) peuvent également être utilisés sous forme de poudres pour poudrage ; une composition comprenant la matière active (50 g) et du talc (950 g) peut également être utilisée ; une composition comprenant la matière active (20 g), de la silice finement divisée (10 g) et du talc (970 g) peut également être utilisée ; ces constituants sont mélangés et broyés et le mélange est appliqué par poudrage.

Comme formes de compositions liquides ou formes destinées à constituer des compositions liquides au cours de l'application, on peut mentionner des solutions, en particulier des concentrés hydrosolubles, des concentrés émulsionnables, des émulsions, des suspensions concentrées, des aérosols ; les poudres mouillables (ou poudres pour poudrage) et les pâtes sont des compositions solides mais qui sont destinées à constituer des compositions liquides au cours de l'application.

Les concentrés émulsionnables ou solubles comprennent le plus souvent 10 à 80 % de matière active ; les émulsions ou solutions prêtes à l'application contiennent quant à elles, 0,001 à 20 % de matière active.

En plus du solvant, les concentrés émulsionnables peuvent contenir, lorsque cela est nécessaire, 2 à 20 % d'additifs convenables tels que les stabilisants, les agents tensio-actifs, les agents accroissant la capacité de pénétration, les inhibiteurs de corrosion, les colorants ou les adhésifs précités.

Des émulsions à n'importe quelle concentration désirée, qui conviennent particulièrement pour l'application à des cultures, peuvent être obtenues à partir de ces concentrés par dilution avec de l'eau.

A titre d'exemple, les compositions de certains concentrés émulsionnables sont présentées :

**Exemple F 8 :**

* Matière active 400 g/l
* Dodécylbenzènesulfonate de métal alcalin 24 g/l
* Produit de condensation oxyde d'éthylène/nonylphénol contenant 10 molécules d'oxyde d'éthylène 16 g/l
* Cyclohexanone 200 g/l
* Solvant aromatique q.s. 1 litre

Suivant une autre formule de concentré émulsionnable, on utilise :

**Exemple F 9 :**

* Matière active 250 g
* Huile végétale époxydée 25 g
* Mélange d'un alkylarylsulfonate, d'un polyéther de glycol et d'alcools gras 100 g
* Diméthylformamide 50 g
* Xylène 575 g

Les suspensions concentrées qui peuvent également être appliquées par pulvérisation sont préparées de manière à obtenir un produit fluide stable qui ne forme pas de dépôts et ces suspensions contiennent habituellement 10 à 75 % de matière active, 0,5 à 15 % d'agents tensioactifs, 0,1 à 10 % d'agents thixotropes, 0 à 10 % d'additifs convenables, tels que des agents antimousse, des inhibiteurs de corrosion, des stabilisants, des agents accroissant la capacité de pénétration et des adhésifs puis, comme véhicule, de l'eau ou un liquide organique dans lequel la matière active est faiblement soluble ou insoluble : certaines matières solides organiques ou certains sels inorganiques peuvent être dissous dans le véhicule afin de mieux éviter la sédimentation ou bien, dans le cas de l'eau, peuvent être dissous à titre d'agents antigel.

A titre d'exemple, une composition de suspension concentrée est présentée :

**Exemple F 10 :**

* Composé N° 1 500 g
* Produit de polycondensation oxyde d'éthylène/ phosphate de tristyrylphénol 50 g
* Produit de polycondensation oxyde d'éthylène/ alkylphénol 50 g

| | | |
|---|---|---|
| * | Polycarboxylate de sodium | 20 g |
| * | Ethylèneglycol | 50 g |
| * | Huile de polysiloxane organique (agent anti-mousse) | 1 g |
| * | Polysaccharide | 1,5 g |
| * | Eau | 327,5 g |

Les dispersions et émulsions aqueuses, par exemple les compositions obtenues par dilution avec de l'eau d'une poudre mouillable ou d'un concentré émulsionnable conforme à la présente invention, entrent dans le cadre général de la présente invention. Les émulsions peuvent être du type eau-dans-huile ou huile-dans-eau et elles peuvent avoir une consistance épaisse telle que celle d'une "mayonnaise".

Il va de soi que la présente invention n'a été décrite qu'à titre explicatif, mais nullement limitatif, et que de nombreuses modifications peuvent y être apportées sans sortir de son cadre.

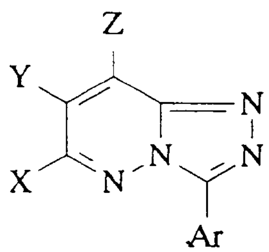

Formule (I)

Numérotation

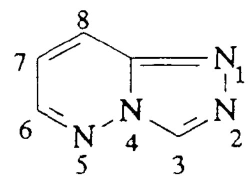

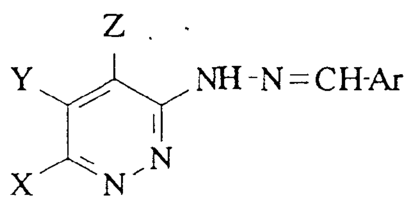

Formule (II)

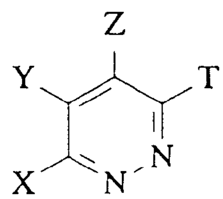

Formule (VIII)

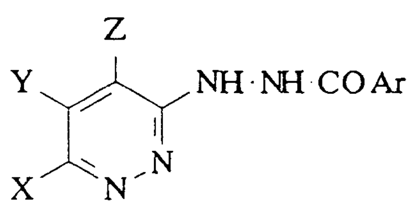

Formule (III)

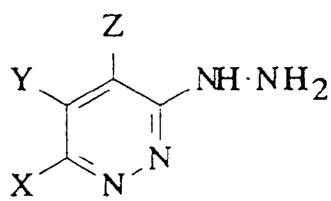

Formule (V)

**Revendications**

1. Dérivés de 1,2,4-triazolo-[4,3-b]pyridazine, caractérisés en ce qu'ils répondent à la formule (I) :

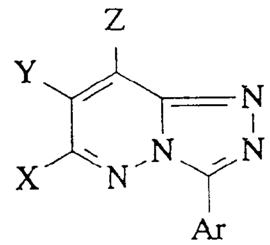

(I)

dans laquelle

X représente un atome d'halogène, un groupe $R^1$ ou un groupe alkoxy, ou bien un atome d'hydrogène ;

Y et Z représentent indépendamment :

l'hydrogène, un halogène, un groupe $R^1$, $-OR^1$, $-SR^1$, $-NR^2R^3$ ou cyano ;

Ar représente :

un groupe phényle facultativement substitué avec un ou plusieurs groupes qui peuvent être identiques ou différents, choisis entre des groupes $R^1$, $-S(O)_mR^1$, $-OR^1$, un groupe cycloalkyle contenant 3 à 6 atomes de carbone, des groupes $-OR^4$, $-OR^5$, $-S(O)_mR^4$, $-NR^2R^4$, $-NR^4COR^1$, $-OCOR^5$, $-S(O)_mR^5$, $-NR^2R^5$, $-NR^5COR^2$, et un atome d'halogène ; ou

un hétérocycle penta- ou hexagonal contenant dans le noyau un ou plusieurs hétéro-atomes choisis entre l'oxygène, le soufre et l'azote, qui est facultativement substitué avec un ou plusieurs groupes qui peuvent être identiques ou différents, choisis entre des groupes $R^1$, $-S(O)_mR^1$, $-OR^1$, un groupe cycloalkyle contenant 3 à 6 atomes de carbone, des groupes $-OR^4$, $-OR^5$, $-S(O)_mR^4$, $-NR^2R^4$, $-NR^4COR^1$, $-OCOR^5$, $-S(O)_mR^5$, $-NR^2R^5$, $-NR^5COR^2$, et un atome d'halogène ;

$R^1$ représente un groupe alkyle à chaîne droite ou ramifiée contenant 1 à 6 atomes de carbone, facultativement substitué avec un ou plusieurs atomes d'halogènes ;

$R^2$ et $R^3$ représentent indépendamment un atome d'hydrogène ou un groupe alkyle à chaîne droite ou ramifiée contenant jusqu'à 4 atomes de carbone, facultativement substitué avec un ou plusieurs atomes d'halogènes ;

$R^4$ représente un groupe phényle facultativement substitué avec un ou plusieurs groupes choisis entre des groupes $R^1$, $-S(O)_mR^1$, $-OR^1$, $-NR^2R^3$, un groupe cycloalkyle contenant 3 à 6 atomes de carbone, et un atome d'halogène ;

$R^5$ représente un hétérocycle penta- ou hexagonal contenant dans le noyau un ou plusieurs hétéro-atomes choisis entre l'oxygène, le soufre et l'azote ;

m a la valeur zéro, 1 ou 2 ; sous réserve que

lorsque X représente le chlore et Y et Z représentent chacun l'hydrogène, Ar représente un groupe autre qu'un groupe phényle non substitué, un groupe 3- ou 4-chloro-, méthyl- ou méthoxy-phényle, un groupe 3-méthoxy-4-méthylphényle, un groupe 2,3,4-triméthoxyphényle ou 3,4-dichlorophényle ; et

lorsque X représente l'hydrogène, un groupe méthyle ou méthoxy et Y et Z représentent l'hydrogène, Ar représente un groupe autre qu'un groupe phényle non substitué ; et leurs sels acceptables en agriculture.

**2.** Composé suivant la revendication 1, caractérisé en ce que :

* X, Y et Z représentent un atome d'hydrogène ou d'halogène ou bien un groupe alkyle, halogénalkyle ou alkoxy,

* Ar représente un groupe phényle, facultativement mono- ou polysubstitué avec un groupe alkyle inférieur, alkoxy inférieur, alkylthio inférieur ou phényle ou un atome d'halogène, de préférence le chlore ou le fluor, ou bien un hétérocycle Het,

* Het représente un hétérocycle penta- ou hexagonal contenant un hétéro-atome tel que le soufre, l'azote ou l'oxygène, cet hétérocycle étant facultativement mono- ou polysubstitué avec un groupe alkyle inférieur, alkoxy inférieur ou alkylthio inférieur ou bien un atome d'halogène, de préférence le chlore ou le fluor.

**3.** Composé suivant la revendication 1 ou 2, caractérisé en ce que Ar est monosubstitué et Het représente un radical thiényle.

**4.** Composé suivant l'une quelconque des revendications 1 à 3, caractérisé en ce que :

un seul des trois radicaux X, Y et Z représente un radical autre qu'un atome d'hydrogène ;

lorsque X, Y et Z représentent des atomes d'halogènes, ils représentent des atomes de chlore,
lorsque X, Y et Z représentent des radicaux hydrocarbonés, au moins partiellement, ces radicaux possèdent de préférence 1 à 4 atomes de carbone.

5. Composé suivant l'une quelconque des revendications 1 à 4, caractérisé en ce que Z représente un groupe $R^1$ ou un atome d'halogène.

6. Composé suivant l'une quelconque des revendications 1 à 4, caractérisé en ce que Z représente un atome d'hydrogène, un atome de chlore ou un groupe méthyle.

7. Composé suivant la revendication 6, caractérisé en ce que X représente un atome d'hydrogène et Y représente un groupe méthyle.

8. Composition herbicide, caractérisée en ce qu'elle comprend comme ingrédient actif une quantité à effet herbicide d'un dérivé de triazolopyridazine de formule (I) suivant l'une quelconque des revendications 1 à 7 ou un de ses sels acceptables en agriculture, en association avec un diluant ou support et/ou agent tensio-actif acceptable en agriculture.

9. Procédé de lutte contre des mauvaises herbes dans un milieu, caractérisé en ce qu'il consiste à appliquer à ce milieu une quantité à effet herbicide d'un dérivé de triazolopyridazine de formule (I) suivant l'une quelconque des revendications 1 à 7 ou d'un de ses sels acceptables en agriculture.

10. Procédé de préparation d'un dérivé de triazolopyridazine de formule (I) suivant la revendication 1 ou 2, caractérisé en ce qu'il consiste à oxyder un composé du type arylidène-2-(pyridaz-3'-yl)hydrazine de formule (II) :

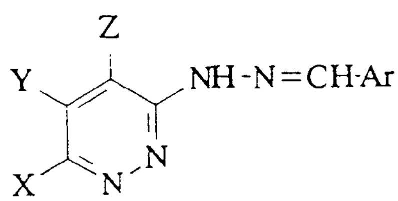

Formule (II)

au moyen d'un agent oxydant, suivant une réaction d'oxydation cyclisante, et, facultativement, à transformer le composé ainsi obtenu en un de ses sels acceptables en agriculture.

11. Procédé suivant la revendication 9, caractérisé en ce que l'agent oxydant est choisi entre des cations métalliques dérivés de métaux ayant plusieurs degrés d'oxydation et étant à un haut degré d'oxydation, ou l'oxygène atmosphérique.

12. Procédé de préparation d'un dérivé de triazolopyridazine de formule (I) suivant la revendication 1 ou 2, caractérisé en ce qu'il consiste à déshydrater un composé du type 3-(aroylhydrazino)pyridazine de formule (III) :

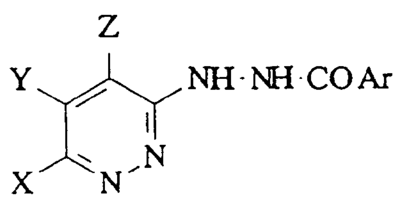

Formule (III)

dans laquelle les différents substituants répondent aux définitions suivant la revendication 1 ou 2 (déshydratation cyclisante) et, facultativement, à transformer le composé ainsi obtenu en un de ses sels acceptables en agriculture.

13. Procédé de préparation d'un dérivé de triazolopyridazine de formule (I) suivant la revendication 1 ou 2, caractérisé en ce qu'il consiste à faire réagir un composé du type hydrazine de formule (V) :

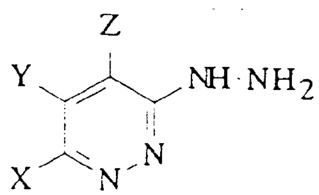

Formule (V)

avec un composé de formule (VI) :

$U_1$-C(=$W_1$)-Ar, conformément à la réaction :

$$(V) + (VI) \rightarrow (I) + U_1H + W_1H_2$$

les différents radicaux dans ces formules (V) et (VI) répondant aux définitions suivant la revendication 1 ou 2 ; et

$W_1$ représente un atome d'oxygène ou un groupe NH,

lorsque $W_1$ représente un atome d'oxygène, $U_1$ représente alors un groupe hydroxyle, alkoxy ou aroyloxy ou bien un atome d'halogène,

lorsque $W_1$ représente un groupe NH, $U_1$ représente alors un groupe alkoxy, alkylthio ou arylalkylthio,

et à transformer facultativement le composé ainsi obtenu en un de ses sels acceptables en agriculture.

14. Procédé de préparation d'un dérivé de triazolopyridazine de formule (I) suivant la revendication 1 ou 2, formule dans laquelle X représente un atome d'hydrogène, caractérisé en ce qu'un composé de formule (I) suivant la revendication 1, formule dans laquelle X représente un atome d'halogène, est hydrogénolysé.

15. Procédé de préparation d'un dérivé de triazolopyridazine de formule (I) suivant la revendication (1) ou (2), formule dans laquelle Z représente un atome de chlore ou de brome et X et Y représentent l'hydrogène ou un groupe $R^1$, caractérisé en ce qu'il consiste à faire réagir un composé de formule (IX) :

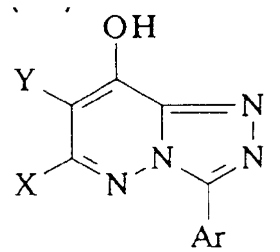

(IX)

dans laquelle X et Y représentent l'hydrogène ou un groupe $R^1$, avec un agent d'halogénation pour le remplacement du groupe hydroxy par un atome de chlore ou de brome, et, facultativement, à transformer le composé ainsi obtenu en un de ses sels acceptables en agriculture.

16. Procédé de préparation d'un dérivé de triazolopyridazine de formule (I) suivant la revendication 1 ou 2, formule dans laquelle Z représente le fluor et X et Y représentent l'hydrogène ou un groupe $R^1$, caractérisé en ce qu'il consiste à faire réagir un composé de formule (I) dans laquelle Z représente le chlore ou le brome et X et Y représentent l'hydrogène ou un groupe $R^1$, avec un sel de formule M-F dans laquelle M représente un métal alcalin ou alcalino-terreux.

17. Procédé de préparation d'arylidène-2-(pyridaz-3'-yl)hydrazines de formule (II) suivant la revendication 10,

caractérisé en ce qu'il consiste à faire réagir une 3-hydrazinopyridazine de formule (V) suivant la revendication 12 avec un aldéhyde de formule Ar-CHO, formules dans lesquelles les symboles X, Y, Z et Ar répondent aux définitions suivant la revendication 1 ou 2.

**18.** Procédé de préparation de 3-(aroylhydrazino)pyridazines de formule (III) suivant la revendication 11, caractérisé en ce qu'il consiste à faire réagir une 3-hydrazinopyridazine de formule (V) suivant la revendication 11 avec des composés de formule (VII) : Ar-CO-$U_2$, formules dans lesquelles les symboles X, Y, Z et Ar répondent aux définitions suivant la revendication 1 ou 2, et $U_2$ répond à l'une des définitions mentionnées pour $U_1$ suivant la revendication 12, conformément au schéma :

$$(V) + (VII) \rightarrow (III) + U_2H$$

**19.** Procédé de préparation de 3-(aroylhydrazino)pyridazines de formule (III) suivant la revendication 11, caractérisé en ce qu'il consiste à faire réagir un dérivé de pyridazine de formule (VIII) :

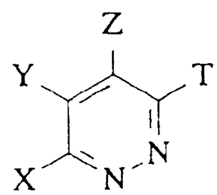

Formule (VIII)

avec un arylhydrazide de formule Ar-CO-NH-$NH_2$, formules dans lesquelles les symboles X, Y, Z et Ar répondent aux définitions suivant la revendication 1 ou 2 et T représente un atome d'halogène.

**20.** Composés caractérisés en ce qu'ils peuvent être utilisés comme intermédiaires pour la préparation des composés suivant la revendication 1 ou 2 et qui répondent à l'une des formules (II), (III), (V) et (VIII) :

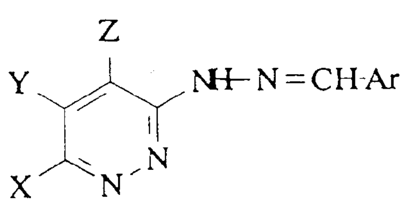

Formule (II)

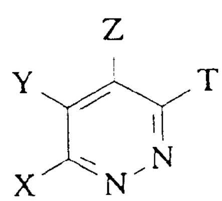

Formule (VIII)

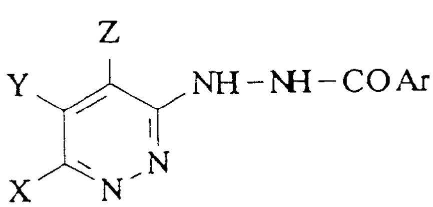

Formule (III)

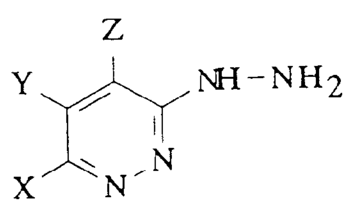

Formule (V)

dans lesquelles les différents symboles répondent aux définitions mentionnées dans la revendication 1, 2 ou 19, sous réserve que :

i) pour des composés de formule (II) :

lorsque X représente un groupe 1-méthyl-1-éthylpropyle et Y et Z représentent chacun l'hydrogène, Ar ne représente pas un groupe 4-n-butoxyphényle, 2,4-, 2,5- ou 3,5-diméthylphényle, 3-bromophényle, 2,4- ou 3,5-dichlorophényle, 2- ou 4-chlorophényle ou 2,4-diméthoxyphényle,

lorsque X, Y et Z représentent chacun un groupe méthyle, Ar ne représente pas un groupe phényle non substitué,

lorsque X et Z représentent chacun un groupe méthyle et Y représente l'hydrogène, Ar ne représente pas un groupe phényle non substitué ou substitué seulement avec le chlore,

lorsque X représente le chlore et Y et Z représentent chacun l'hydrogène, Ar ne représente pas un groupe phényle non substitué ou substitué seulement avec le chlore ou un groupe méthoxy ;

ii) pour des composés de formule (V)

lorsque Y et Z représentent chacun un groupe méthyle, X ne représente pas le chlore ; et

iii) pour des composés de formule (VIII) :

lorsque T représente le chlore et Y et Z représentent chacun un groupe méthyle, X ne représente pas l'hydrogène ou le chlore,

lorsque Y et Z représentent chacun l'hydrogène, X ne représente pas un groupe méthyle, butyle, trichlorométhyle ou un halogène,

lorsque T et Y représentent chacun le chlore, X et Z représentent des groupes autres que le chlore.